# EUROPEAN PATENT APPLICATION

(11) **EP 4 613 731 A1**
(43) Date of publication of application: **10.09.2025**
(21) Application number: 25162046.4
(22) Date of filing: 09.03.2022
(51) Int. Cl.: C07C 43/12, C07C 43/17

(54) **HALOETHER, METHOD FOR PRODUCING SAME, VINYL ETHER, AND METHOD FOR PRODUCING SAME**

(30) Priority: 09.03.2021 JP 2021037781
(62) Divisional of application: 22767208.6
(71) Applicant: DAIKIN INDUSTRIES, LTD., Osaka-shi, Osaka 530-0001 (JP)
(72) Inventor: MATSUMOTO, Akane, Osaka-shi, Osaka, 530-0001 (JP); HOSHIYA, NaoyukI, Osaka-shi, Osaka, 530-0001 (JP); YAMAUCHI, Akiyoshi, Osaka-shi, Osaka, 530-0001 (JP); KISHIKAWA, Yosuke, Osaka-shi, Osaka, 530-0001 (JP)
(74) Representative: Hoffmann Eitle

(57) **Abstract**

An object of the present invention is to provide a vinyl ether, a simple method for producing the vinyl ether, a haloether used for producing the vinyl ether, and a method for producing the vinyl ether.

The object can be achieved by producing a haloether by a method comprising step 1a of allowing a compound represented by formula (1):

R¹O⁻ Y⁺ (1)

wherein
Y⁺ represents a cation and R¹ is an organic group,
to react, in the presence of a halogenating agent, with a compound represented by formula (2a): 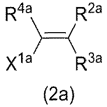 wherein
R^{2a} and R^{3a} each independently represent H or an alkyl group,
R^{4a} represents H, a halogen, or an organic group,
and X^{1a} represents a halogen other than F.

## Description

### Technical Field

The present disclosure relates to a haloether, a method for producing the haloether, a vinyl ether, and a method for producing the vinyl ether.

### Background Art

The conventional synthesis method of fluoro vinyl ether requires multiple steps. Specifically, after a fluoroalkoxide salt was synthesized by reacting an acid fluoride with a fluoride salt, 1) a substitution reaction with ethyl bromfluoroacetate, 2) a reduction reaction using LiAlH₄, 3) a tosylation reaction using tosyl chloride and pyridine, and 4) desorption reaction of p-toluene sulfonic acid with lithium (bistrimethylsilyl)amide (LiHMDS) were performed to synthesize a fluoro vinyl ether (Non-patent Literature 1).

### Citation List

### Non-patent Literature

NPL 1: Yagupolskii et al., Journal of Fluorine Chemistry, 2015, vol. 179, pp. 134-141

### Summary of Invention

### Technical Problem

The synthesis method of a fluoro vinyl ether in Non-patent Literature 1 comprises multiple steps, and there is room for improvement in terms of simplification of the synthetic method.

The present disclosure relates to a vinyl ether, a simple method for producing the vinyl ether, a haloether used for producing the vinyl ether, and a method for producing the vinyl ether.

### Solution to Problem

The present disclosure includes the following aspects.
Item 1. A method for producing a compound represented by formula (4a): 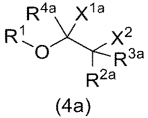 wherein
   - R¹: represents an organic group,
   - R^{2a} and R^{3a}: each independently represent H or an alkyl group,
   - R^{4a}: represents H, a halogen, or an organic group,
   - X^{1a}: represents a halogen other than F, and
   - X²: represents a halogen,
   the method comprising step 1a of allowing a compound represented by formula (1):

   R¹O⁻ Y⁺ (1)

   wherein
   - Y⁺: represents a cation and R¹ is as defined above,
   to react, in the presence of a halogenating agent, with a compound represented by formula (2a): 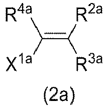 wherein symbols in the formula are as defined above.
Item 2. The production method according to Item 1, wherein the halogenating agent is a compound represented by formula (3a): X²-Z (3a)
   wherein
   - X²: represents a halogen,
   - Z: represents a halogen or NZ¹Z²,
   - Z¹ and Z²: each independently represent an organic group, or Z¹ and
   - Z²: are bound together to form a ring, or
   - a: compound represented by formula (3a'): 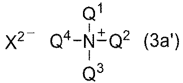
   wherein
   - X²: represents a halogen,
   - Q¹ to Q⁴: each independently represent H or an organic group, and
   any two of Q¹ to Q⁴ are optionally bound together to form a ring.
Item 3. The production method according to Item 1 or 2, wherein Y⁺ represents a metal ion or a quaternary ammonium ion.
Item 4. The production method according to any one of Items 1 to 3, wherein R¹ represents a fluoroalkyl group optionally having one or more substituents, and the fluoroalkyl group optionally contains a heteroatom between carbon atoms.
Item 5. The production method according to any one of Items 1 to 4, wherein R¹ represents a C₁₋C₁₀ fluoroalkyl group optionally having one or more substituents, and the C₁₋C₁₀ fluoroalkyl group optionally contains a heteroatom between carbon atoms.
Item 6. The production method according to any one of Items 1 to 5, wherein R^{4a} is F or a fluoroalkyl group.
Item 7. The production method according to any one of Items 1 to 6, wherein R^{2a} and R^{3a} each independently represent H or a C₁₋C₃ alkyl group.
Item 8. The production method according to any one of Items 1 to 7, wherein step 1a is performed in the absence of a catalyst.
Item 9. A method for producing a compound represented by formula (4b): 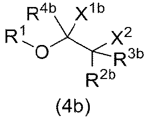 wherein
   R¹ represents an organic group,
   R^{2b} and R^{3b} each independently represent H, a halogen, or an alkyl group optionally having one or more substituents,
   R^{4b} represents H, a halogen, or an organic group, and
   X^{1b} and X² each independently represent a halogen, the method comprising step 1b of allowing a compound represented by formula (1):

      R¹O⁻ Y⁺ (1)

      wherein
      Y⁺ represents a cation and R¹ is as defined above,
      to react, in the presence of a halogenating agent and a catalyst, with a compound represented by formula (2b): 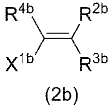
      wherein symbols in the formula are as defined above.
Item 10. The production method according to Item 9, wherein when R^{4b} represents F or a fluoroalkyl group, X^{1b} represents a halogen other than F.
Item 11. The production method according to Item 9 or 10, wherein the halogenating agent is a compound represented by formula (3a):

   X²-Z (3a)

   wherein
   X² represents a halogen,
   Z represents a halogen or NZ¹Z²,
   Z¹ and Z² each independently represent an organic group, or Z¹ and
   Z² are bound together to form a ring, or
   a compound represented by formula (3a'): 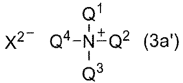 wherein
      X² represents a halogen,
      Q¹ to Q⁴ each independently represent H or an organic group, and
      any two of Q¹ to Q⁴ are optionally bound together to form a ring.
Item 12. The production method according to any one of Items 9 to 11, wherein the catalyst is a Lewis base.
Item 13. The production method according to any one of Items 9 to 12, wherein the catalyst is a Lewis base represented by formula (3b):

   L¹- (Z³) ₙ (3b)

   wherein
   L¹ represents O, N, (S) ₘ, P, or (Se)ₖ,
   m and k are 1 or 2,
   n represents a number corresponding to the valence of L¹,
   Z³s each independently represent an organic group, and when n represents 2 or more, any two Z³s are optionally bound together to form a ring,
   or a Lewis base represented by formula (3c):

      L²=Z⁴ (3c)

      wherein
      L² represents O, S, or Se,
      Z⁴ represents P(Z⁵)₃ or C(Z⁶)₂, Z⁵s each independently represent an organic group, and any two Z⁵s are optionally bound together to form a ring, and Z⁶s each independently represent an organic group, and any two Z⁶s are optionally bound together to form a ring.
Item 14. The production method according to any one of Items 9 to 13, wherein Y⁺ represents a metal ion or a quaternary ammonium ion.
Item 15. The production method according to any one of Items 9 to 14, wherein R¹ represents a fluoroalkyl group optionally having one or more substituents, and the fluoroalkyl group optionally contains a heteroatom between carbon atoms.
Item 16. The production method according to any one of Items 9 to 15, wherein R^{2b} and R^{3b} each independently represent H, a halogen, an alkyl group, or a fluoroalkyl group.
Item 17. The production method according to any one of Items 9 to 16, wherein R^{4b} represents a halogen or a fluoroalkyl group.
Item 18. A compound represented by formula (4a): 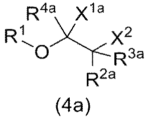 wherein
   R¹ represents an organic group,
   R^{2a} and R^{3a} each independently represent H or an alkyl group,
   R^{4a} represents H, a halogen, or an organic group,
   X^{1a} represents a halogen other than F, and
   X² represents a halogen.
Item 19. A composition comprising a compound represented by formula (4b):
   wherein 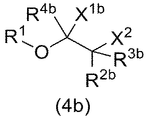
   R¹ represents an organic group,
   R^{2b} and R^{3b} each independently represent H, a halogen, or an alkyl group optionally having one or more substituents,
   R^{4b} represents H, a halogen, or an organic group,
   X^{1b} and X² each independently represent a halogen; and
   a halide ion.
Item 20. A composition comprising a compound represented by formula (4b): 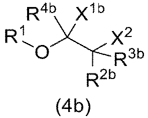 wherein
   R¹ represents an organic group,
   R^{2b} and R^{3b} each independently represent H, a halogen, or an alkyl group optionally having one or more substituents,
   R^{4b} represents H, a halogen, or an organic group, and
   X^{1b} and X² each independently represent a halogen; and water.
Item 21. The composition according to Item 19 or 20, wherein when R^{4b} represents F or a fluoroalkyl group, X^{1b} represents a halogen other than F.
Item 22. A method for producing a compound represented by formula (5a): 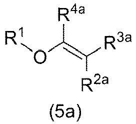 wherein
   R¹ represents an organic group,
   R^{2a} and R^{3a} each independently represent H or an alkyl group,
   R^{4a} represents H, a halogen, or an organic group,
   the method comprising step 2a of allowing a reducing agent to react with a compound represented by formula (4a): 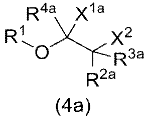 wherein
      R¹, R^{2a}, R^{3a}, and R^{4a} are as defined above,
      X^{1a} represents a halogen other than F, and
      X² represents a halogen.
Item 23. The production method according to Item 22, wherein R¹ represents a fluoroalkyl group optionally having one or more substituents, and the fluoroalkyl group optionally contains a heteroatom between carbon atoms.
Item 24. The production method according to Item 22 or 23, wherein R¹ represents a C₁₋C₁₀ fluoroalkyl group optionally having one or more substituents, and the C₁₋C₁₀ fluoroalkyl group optionally contains a heteroatom between carbon atoms.
Item 25. The production method according to any one of Items 22 to 24, wherein R^{4a} represents F or a fluoroalkyl group.
Item 26. The production method according to any one of Items 22 to 25, wherein the reducing agent is an organometallic reagent.
Item 27. The production method according to any one of Items 22 to 25,
   wherein the reducing agent is at least one member selected from the group consisting of
   (i) transition metals, alkali metals, and alkaline earth metals;
   (ii) metal pairs of transition metals; and
   (iii) mixtures of transition metals with acids or metal salts.
Item 28. The production method according to any one of Items 22 to 25, wherein the reducing agent is a phosphorus-containing compound.
Item 29. A method for producing a compound represented by formula (5a): 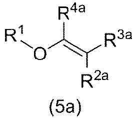 wherein
   R¹ represents an organic group,
   R^{2a} and R^{3a} each independently represent H or an alkyl group, and
   R^{4a} represents H, a halogen, or an organic group,
   the method comprising a step of producing a compound represented by formula (4a): 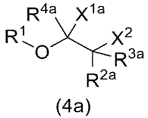 wherein
      R¹, R^{2a}, R^{3a}, R^{4a}, and X^{1a} are as defined above, and
      X² represents a halogen, comprising
      step 1a of allowing a compound represented by formula (1):

         R¹O⁻ Y⁺ (1)

         wherein
         Y⁺ represents a cation and R¹ is as defined above,
         to react, in the presence of a halogenating agent, with a compound represented by formula (2a): 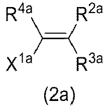 wherein
            R^{2a}, R^{3a}, and R^{4a} are as defined above,
            X^{1a} represents a halogen other than F, and step 2a of allowing a reducing agent to react with the compound represented by formula (4a).
Item 30. A method for producing a compound represented by formula (5b): 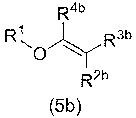 wherein
   R¹ represents an organic group,
   R^{2b} and R^{3b} each independently represent H, a halogen, or an alkyl group optionally having one or more substituents, and
   R^{4b} represents H, a halogen, or an organic group,
   the method comprising a step of producing a compound represented by formula (4b): 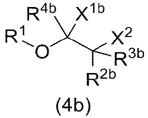 wherein
      R¹, R^{2b}, R^{3b}, R^{4b}, and X^{1b} are as defined above, and
      X² represents a halogen, comprising
      step (1b) of allowing a compound represented by formula (1):

         R¹O⁻ Y⁺ (1)

         wherein
         Y⁺ represents a cation and R¹ is as defined above,
         to react, in the presence of a halogenating agent and a catalyst,
         with a compound represented by formula (2b): 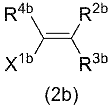 wherein
            R^{2b}, R^{3b}, and R^{4b} are as defined above, and
            X^{1b} represents a halogen, and
            step 2b of allowing a reducing agent to react with the compound represented by formula (4b).
Item 31. The production method according to Item 30, wherein when R^{4b} represents F or a fluoroalkyl group, X^{1b} represents a halogen other than F.
Item 32. A compound represented by formula (5c): 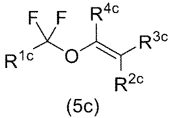 wherein
   R^{1c} represents a fluoroalkyl group having two or more carbon atoms and optionally having one or more substituents, or a fluoroalkoxy group having one or more carbon atoms and optionally having one or more substituents, wherein the fluoroalkyl group or the fluoroalkoxy group optionally contains a heteroatom between carbon atoms,
   R^{2c} and R^{3c} each independently represent H or an alkyl group, and
   R^{4c} represents F.
Item 33. A composition comprising a compound represented by formula (5b): 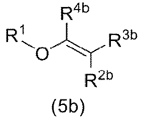 wherein
   R¹ represents an organic group,
   R^{2b} and R^{3b} each independently represent H, a halogen, or an alkyl group optionally having one or more substituents,
   R^{4b} represents H, a halogen, or an organic group; and a halide ion.
Item 34. A composition comprising a compound represented by formula (5b): 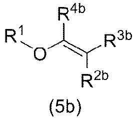 wherein
   R¹ represents an organic group,
   R^{2b} and R^{3b} each independently represent H, a halogen, or an alkyl group optionally having one or more substituents,
   R^{4b} represents H, a halogen, or an organic group; and
   water.

### Advantageous Effects of Invention

The present disclosure can provide a vinyl ether, a simple method for producing the vinyl ether, a haloether used in the method for producing a vinyl ether, and a method for producing the haloether.

### Description of Embodiments

The above overview of the present disclosure is not intended to describe each of the disclosed embodiments or all of the implementations of the present disclosure.

The following description of the present disclosure more specifically exemplifies the embodiments of the examples.

In several parts of the present disclosure, guidance is provided through examples, and these examples can be used in various combinations.

In each case, the group of examples can function as a non-exclusive and representative group.

All publications, patents, and patent applications cited herein are incorporated herein by reference in their entirety.

### 1. Terms

Unless otherwise specified, the symbols and abbreviations in the present specification can be understood in the context of the present specification in the meanings commonly used in the technical field to which the present disclosure belongs.

In the present specification, the terms "comprise" and "contain" are used with the intention of including the terms "consisting essentially of" and "consisting of."

Unless otherwise specified, the steps, treatments, or operations described in the present specification can be performed at room temperature.

In the present specification, room temperature can refer to a temperature within the range of 10 to 40°C.

In the present specification, the phrase "Cₙ₋ₘ" (n and m are each a number) indicates that the number of carbon atoms is n or more and m or less, as can be generally understood by a person skilled in the art.

In the present specification, the phrase "compound represented by formula (N)" can be referred to as "compound (N)."

In the present specification, the "organic group" refers to a group containing one or more carbon atoms.

Examples of the organic group may include
a hydrocarbon group optionally having one or more substituents, an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents,
a heteroaryl group optionally having one or more substituents,
a cyano group,
an aldehyde group,

   R^{r}O-,

   R^{r}S-,

   R^{r}NH- ,

   (R^{r})₂N-,

   R^{r}CO-,

   R^{r}COO-,

   R^{r}SO₂-,

   R^{r}OCO-, and

   R^{r}OSO₂-
wherein, R^{r} is independently
an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents, or
a heteroaryl group optionally having one or more substituents).

In the present specification, unless otherwise specified, examples of the hydrocarbon group may include an alkyl group, an alkenyl group, an alkynyl group, a cycloalkyl group, a cycloalkenyl group, a cycloalkadienyl group, an aryl group, an aralkyl group, and a group that is a combination of these groups.

In the present specification, unless otherwise specified, examples of the alkyl group may include linear or branched C₁₋C₁₂ alkyl groups, such as methyl, ethyl, propyl, isopropyl, butyl, isobutyl, sec-butyl, tert-butyl, pentyl, isopentyl, neopentyl, hexyl, heptyl, octyl, nonyl, and decyl.

In the present specification, unless otherwise specified, examples of the alkenyl group may include linear or branched C₂-C₁₀ alkenyl groups, such as vinyl, 1-propen-1-yl, 2-propen-1-yl, isopropenyl, 2-buten-1-yl, 4-penten-1-yl, and 5-hexen-1-yl.

In the present specification, unless otherwise specified, examples of the alkynyl group may include linear or branched C₂-C₁₀ alkynyl groups, such as ethynyl, 1-propyn-1-yl, 2-propyn-1-yl, 4-pentyn-1-yl, and 5-hexyn-1-yl.

In the present specification, unless otherwise specified, examples of the cycloalkyl group may include C₃-C₇ cycloalkyl groups, such as cyclopropyl, cyclobutyl, cyclopentyl, cyclohexyl, and cycloheptyl.

In the present specification, unless otherwise specified, examples of the cycloalkenyl group may include C₃-C₇ cycloalkenyl groups, such as cyclopropenyl, cyclobutenyl, cyclopentenyl, cyclohexenyl, and cycloheptenyl.

In the present specification, unless otherwise specified, examples of the cycloalkadienyl group may include C₄-C₁₀ cycloalkadienyl groups, such as cyclobutadienyl, cyclopentadienyl, cyclohexadienyl, cycloheptadienyl, cyclooctadienyl, cyclononadienyl, and cyclodecadienyl.

In the present specification, unless otherwise specified, the aryl group may be monocyclic, bicyclic, tricyclic, or tetracyclic.

In the present specification, unless otherwise specified, the aryl group may be a C₆-C₁₈ aryl group.

In the present specification, unless otherwise specified, examples of the aryl group may include phenyl, 1-naphthyl, 2-naphthyl, 2-biphenyl, 3-biphenyl, 4-biphenyl, and 2-anthryl.

In the present specification, unless otherwise specified, examples of the aralkyl group may include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, and 4-biphenylylmethyl.

In the present specification, unless otherwise specified, the non-aromatic heterocyclic group may be monocyclic, bicyclic, tricyclic, or tetracyclic.

In the present specification, unless otherwise specified, the non-aromatic heterocyclic group may be, for example, a non-aromatic heterocyclic group containing, in addition to carbon, 1 to 4 heteroatoms selected from oxygen, sulfur, and nitrogen as a ring-constituting atom.

In the present specification, unless otherwise specified, the non-aromatic heterocyclic group may be saturated or unsaturated.

In the present specification, unless otherwise specified, examples of the non-aromatic heterocyclic group may include tetrahydrofuryl, oxazolidinyl, imidazolinyl (e.g., 1-imidazolinyl, 2-imidazolinyl, and 4-imidazolinyl), aziridinyl (e.g., 1-aziridinyl and 2-aziridinyl), azetidinyl (e.g., 1-azetidinyl and 2-azetidinyl), pyrrolidinyl (e.g., 1-pyrrolidinyl, 2-pyrrolidinyl, and 3-pyrrolidinyl), piperidinyl (e.g., 1-piperidinyl, 2-piperidinyl, and 3-piperidinyl), azepanyl (e.g., 1-azepanyl, 2-azepanyl, 3-azepanyl, and 4-azepanyl), azocanyl (e.g., 1-azocanyl, 2-azocanyl, 3-azocanyl, and 4-azocanyl), piperazinyl (e.g., 1,4-piperazin-1-yl and 1,4-piperazin-2-yl), diazepinyl (e.g., 1,4-diazepin-1-yl, 1,4-diazepin-2-yl, 1,4-diazepin-5-yl, and 1,4-diazepin-6-yl), diazocanyl (e.g., 1,4-diazocan-1-yl, 1,4-diazocan-2-yl, 1,4-diazocan-5-yl, 1,4-diazocan-6-yl, 1,5-diazocan-1-yl, 1,5-diazocan-2-yl, and 1,5-diazocan-3-yl), tetrahydropyranyl (e.g., tetrahydropyran-4-yl), morpholinyl (e.g., 4-morpholinyl), thiomorpholinyl (e.g., 4-thiomorpholinyl), 2-oxazolidinyl, dihydrofuryl, dihydropyranyl, dihydroquinolyl, and the like.

In the present specification, unless otherwise specified, examples of the heteroaryl group may include monocyclic aromatic heterocyclic groups (e.g., 5- or 6-membered monocyclic aromatic heterocyclic groups) and aromatic condensed heterocyclic groups (e.g., 5- to 18-membered aromatic condensed heterocyclic groups).

In the present specification, unless otherwise specified, examples of the 5- or 6-membered monocyclic aromatic heterocyclic groups may include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-4-yl and 1,2,4-triazol-3-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), pyrazinyl, and the like.

In the present specification, unless otherwise specified, examples of the 5- to 18-membered aromatic condensed heterocyclic groups may include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl (e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl).

In the present specification, examples of R^{r}O- may include alkoxy (e.g., C₁₋C₁₀ alkoxy, such as methoxy, ethoxy, and propoxy), cycloalkoxy (e.g., C₃-C₇ cycloalkoxy, such as cyclopentoxy and cyclohexoxy), aryloxy (e.g., C₆-C₁₈ aryloxy, such as phenoxy and naphthoxy), and aralkyloxy (e.g., C₇-C₁₉ aralkyloxy, such as benzyloxy and phenethyloxy).

In the present specification, examples of "R^{r}S-" include alkoxylthio (e.g., C₁₋C₁₀ alkylthio, such as methylthio, ethylthio, and propylthio), cycloalkylthio (e.g., C₃-C₇ cycloalkylthio, such as cyclopentylthio and cyclohexylthio), arylthio (e.g., C₆-C₁₈ arylthio, such as phenylthio and naphthylthio), and aralkylthio (e.g., C₇-C₁₉ aralkylthio, such as benzylthio and phenethylthio).

In the present specification, examples of "R^{r}NH-" include monoalkylamino (e.g., mono C₁₋C₁₀ alkylamino, such as monomethylamino, monoethylamino, and monopropylamino), monocycloalkylamino (e.g., mono C₃-C₇ cycloalkylamino, such as monocyclopentylamino and monocyclohexylamino), monoarylamino (e.g., mono C₆-C₁₈ arylamino, such as monophenylamino and mononaphthylamino), and monoaralkylamino (e.g., mono C₇-C₁₉ aralkylamino, such as monobenzylamino and monophenethylamino).

In the present specification, examples of "(R^{r})₂N-" include dialkylamino (e.g., diC₁₋C₁₀ alkylamino, such as dimethylamino, ethylmethylamino, and diethylamino), N-aryl-N-alkylamino (e.g., N-C₆₋C₁₈ aryl-N-C₁₋C₁₀ alkylamino, such as N-phenyl-N-methylamino), and diarylamino (e.g., diC₆₋C₁₈ arylamino, such as diphenylamino).

In the present specification, examples of R^{r}CO- may include alkylcarbonyl (e.g., (C₁-C₁₀ alkyl)carbonyl, such as acetyl, propionyl, and butyryl), cycloalkylcarbonyl (e.g., (C₃-C₇ cycloalkyl)carbonyl, such as cyclopentanoyl and cyclohexanoyl), arylcarbonyl (e.g., (C₆-C₁₈ aryl)carbonyl, such as benzoyl and naphthoyl), and aralkylcarbonyl (e.g., (C₇-C₁₉ aralkyl)carbonyl, such as benzylcarbonyl and phenethylcarbonyl).

In the present specification, examples of R^{r}COO- may include alkylcarbonyloxy (e.g., (C₁-C₁ₒ alkyl)carbonyloxy, such as acetyloxy, propionyloxy, and butyryloxy), cycloalkylcarbonyloxy (e.g., (C₃-C₇ cycloalkyl)carbonyloxy, such as cyclopentanoyloxy and cyclohexanoyloxy), arylcarbonyloxy (e.g., (C₆-C₁₈ aryl)carbonyloxy, such as benzoyloxy and naphthoyloxy), and aralkylcarbonyloxy (e.g., (C₇-C₁₉ aralkyl)carbonyloxy, such as benzylcarbonyloxy and phenethylcarbonyloxy).

In the present specification, examples of R^{r}SO₂- may include alkylsulfonyl (e.g., C₁₋C₁₀ alkylsulfonyl, such as methylsulfonyl, ethylsulfonyl, and propylsulfonyl), cycloalkylsulfonyl (e.g., C₄-C₈ cycloalkylsulfonyl, such as cyclopentylsulfonyl and cyclohexylsulfonyl), arylsulfonyl (e.g., C₆-C₁₈ arylsulfonyl, such as phenylsulfonyl and naphthylsulfonyl), and aralkylsulfonyl (e.g., C₇-C₁₉ aralkylsulfonyl, such as benzylsulfonyl and phenethylsulfonyl).

In the present specification, examples of R^{r}OCO- may include alkoxycarbonyl (e.g., C₁₋C₁₀ alkoxy)carbonyl, such as methoxycarbonyl, ethoxycarbonyl, and propoxycarbonyl), cycloalkoxycarbonyl (e.g., (C₃-C₇ cycloalkoxy)carbonyl, such as cyclopentoxycarbonyl and cyclohexoxycarbonyl), aryloxycarbonyl (e.g., (C₆-C₁₈ aryloxy)carbonyl, such as phenoxycarbonyl and naphthoxycarbonyl), and aralkyloxycarbonyl (e.g., (C₇-C₁₉ aralkyloxy)carbonyl, such as benzyloxycarbonyl and phenethyloxycarbonyl.)

In the present specification, examples of R^{r}OSO₂- may include alkoxysulfonyl (e.g., C₁₋C₁₀ alkoxysulfonyl, such as methoxysulfonyl, ethoxysulfonyl, and propoxysulfonyl), cycloalkoxysulfonyl (e.g., C₃-C₇ cycloalkoxysulfonyl, such as cyclopentoxysulfonyl and cyclohexoxysulfonyl), aryloxysulfonyl (e.g., C₆-C₁₈ aryloxysulfonyl, such as phenoxysulfonyl and naphthoxysulfonyl), and aralkyloxysulfonyl (e.g., C₇-C₁₉ aralkyloxysulfonyl, such as benzyloxysulfonyl and phenethyloxysulfonyl).

In the present specification, examples of the substituents in the "hydrocarbon group optionally having one or more substituents," "alkyl group optionally having one or more substituents," "alkenyl group optionally having one or more substituents," "alkynyl group optionally having one or more substituents," "cycloalkyl group optionally having one or more substituents," "cycloalkenyl group optionally having one or more substituents," "cycloalkadienyl group optionally having one or more substituents," "aryl group optionally having one or more substituents," "aralkyl group optionally having one or more substituents," "non-aromatic heterocyclic group optionally having one or more substituents," and "heteroaryl group optionally having one or more substituents" may include a halo group, a nitro group, a cyano group, an oxo group, a thioxo group, a sulfo group, a sulfamoyl group, a sulfinamoyl group, a sulfenamoyl group, R^{r}O-, R^{r}S-, R^{r}CO-, R^{r}COO-, R^{r}SO₂-, R^{r}OCO-, and R^{r}OSO₂- (in these formulas, R^{r} is as defined above).

Of these substituents, examples of the halo group may include fluoro, chloro, bromo, and iodo.

The number of substituents may be within the range of 1 to the maximum substitutable number (e.g., 1, 2, 3, 4, 5, or 6).

### 2. Method for Producing the Compound Represented by Formula (4a)

In one embodiment, the method for producing the compound represented by formula (4a) comprises step 1a of allowing the compound represented by formula (1) to react with the compound represented by formula (2a) in the presence of a halogenating agent.

### 2-1. Compound Represented by Formula (1)

In formula (1), R¹ is not limited as long as it can form an oxide. R¹ is preferably a hydrocarbon group (such a hydrocarbon group optionally contains a heteroatom between carbon atoms) optionally having one or more substituents, more preferably an alkyl group (the alkyl group optionally contains a heteroatom between carbon atoms) optionally having one or more substituents. The "one or more substituents" that can be contained in the hydrocarbon group and alkyl group preferably include a halo group, and even more preferably include a fluoro group. Specifically, R¹ preferably represents a haloalkyl group (the haloalkyl group optionally contains a heteroatom between carbon atoms) optionally having one or more substituents, and more preferably a fluoroalkyl group (the fluoroalkyl group optionally contains a heteroatom between carbon atoms) optionally having one or more substituents. The fluoroalkyl group may be a perfluoroalkyl group. The "one or more substituents" that can be contained in the haloalkyl group and the fluoroalkyl group preferably include a nitrile group, an ester group (e.g., R^{r}OCO-), a formyl group, and a sulfonyl-containing group (e.g. R^{r}OSO₂-). The "heteroatom" that can be included between carbon atoms of the hydrocarbon group, alkyl group, haloalkyl group, and fluoroalkyl group include, but are not limited to, an oxygen atom, a sulfur atom, and a nitrogen atom. The number of carbon atoms in the hydrocarbon, alkyl group, haloalkyl group, and fluoroalkyl group is not limited as long as it is at least 1. For example, the number of carbon atoms is in the range of 1 to 12, preferably in the range of 1 to 9, more preferably in the range of 1 to 6, and most preferably in the range 1 to 4.

Examples of the "fluoroalkyl group optionally having one or more substituents (the fluoroalkyl group optionally contains a heteroatom between carbon atoms)" include
CF₃-,
CH₃-CF₂-,
CHF₂-CH₂-,
CF₃-CH₂-,
CF₃-CF₂-,
CF₃-CF₂-CH₂-,
CF₃-CF₂-CF₂-,
(CF₃)₂CF-,
CF₃-O-CF₂-,
CF₃-O-CF (CF₃) -,
CF₃-CF₂-CF₂-CF₂-,
CF₃-CF₂-CF (CF₃) -CF₂-,
CF₃-O-CH₂-CH₂-,
CF₃-O-CH(CF₃) -CH₂-,
CF₃-O-CF₂-CF₂-,
(CF₃CF₂) (CF₂)CF-,
(CF₃)₃C-,
CF₃-CF₂-O-CF₂-,
CF₃-CF₂-CF₂-CF₂-CF₂-,
CF₃-CF₂-O-CF₂-CF₂-,
CF₃-O-CF₂-O-CF₂-,
CF₃-CF₂-CF₂-CF₂-CF₂-CF₂-,
CF₃-CF₂-CF₂-O-CH₂-CF₂-,
CF₃-CF₂-CF₂-O-CF₂-CF₂-,
CF₃-CF₂-CF₂-CF₂-CF₂-CF₂-CF₂-,
CF₃-CF₂-CF₂-CF₂-CF₂-CF₂-CF₂-CF₂-,
CHF₂-CF₂-CF₂-CF₂-CF₂-CF₂-CF₂-CH₂-
CF₃-CF (CF₃) -CF₂-,
CF₃-CF₂-CF (CF₃) -,
CF₃-CF₂-CF₂-O-CF(CF₃)-CF₂-,
CF₃-CF₂-CF₂-O-CF (CF₃) -CF₂-O-CF (CF₃) -CF₂-,
CF₃-CF₂-CF₂-O- [CF (CF₃) -CF₂-O-1 ₂-CF (CF₃) -CF₂-
and like fluoro C₁₋C₁₀ alkyl groups, fluoro C₁₋C₃ alkoxy fluoro C₁₋C₃ alkyl groups, fluoro C₁₋C₃ alkoxy fluoro C₁₋C₃ alkoxy fluoro C₁₋C₃ alkyl groups, fluoro C₁₋C₃ alkoxy fluoro C₁₋C₃ alkoxy fluoro C₁₋C₃ alkoxy fluoro C₁₋C₃ alkyl groups, etc.

In formula (1), Y⁺ is not limited as long as it can be a counterion of R¹O⁻. Y⁺ represents, for example, a metal ion or an ion represented by formula (1a): 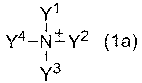 wherein
Y¹ to Y⁴ each independently represent H or an organic group, and any two or three of Y¹ to Y⁴ are optionally bound together to form a ring. Examples of ions represented by formula (1a) include imidazolium ions, pyridinium ions, quinolinium ions, triazinium ions, and ammonium ions.

Metal ions include monovalent or divalent metal ions, and specific examples include lithium, sodium, potassium, cesium, and silver.

In formula (1a), Y¹ to Y⁴ are not limited as long as they can form nitrogen-containing ions. Y¹ to Y⁴ each preferably represent H or a hydrocarbon group optionally having one or more substituents, more preferably H, an alkyl group optionally having one or more substituents, a cycloalkyl group optionally having one or more substituents, an aryl group optionally having one or more substituents, or an aralkyl group optionally having one or more substituents, and alkyl group optionally having one or more substituents, and particularly preferably a C₁₋C₄ alkyl group optionally having one or more substituents. The "substituent" that can be contained in the hydrocarbon group, alkyl group, cycloalkyl group, aryl group, and aralkyl group includes, but is not limited to, a halo group, a hydroxyl group, and a mercapto group.

The ring formed by binding any two of Y¹ to Y⁴ to each other includes, but is not limited to, five- or six-membered rings, such as pyrrolidine, piperidine, and morpholine.

Y⁺ preferably represents a metal ion or a quaternary ammonium ion, such as an ion in which all of the Y¹ to Y⁴ are other than H in formula (1a). The metal ion is, for example, a monovalent or divalent metal ion, preferably a monovalent or divalent metal ion selected from the group consisting of alkali metals, alkaline earth metals, and transition metal groups, and even more preferably lithium, sodium, potassium, cesium, or silver.

The compound represented by formula (1) may be used directly as it is, or the compound represented by formula (1) may be generated in a reaction system from the compound represented by formula (1') or formula (1''):

R¹-COF (1')

R¹-O-CH₃ (1")

wherein R¹ is as defined above, with the proviso that in formula (1''), R¹ represents R^{1'}-CF₂, and R^{1'} represents an organic group.

Specifically, the compound represented by formula (1) can be produced by reacting the compound represented by formula (1') with a fluorinating agent. Specifically, instead of directly using the compound represented by formula (1) as a reactant in steps 1a and 1b, the compound represented by formula (1') and the fluorinating agent can be used. The fluorinating agent is, for example, the compound represented by F⁻Y⁺ (Y⁺ is as defined above), preferably an alkali metal fluoride, such as potassium fluoride, rubidium fluoride, and cesium fluoride, and a tetra C₁₋C₄ alkylammonium fluoride, such as tetramethylammonium fluoride and tetraethylammonium fluoride.

The compound represented by formula (1) can also be produced by reacting the compound represented by formula (1") with an amine. Specifically, instead of directly using the compound represented by formula (1), the compound represented by formula (1') and an amine can be used as reactants in steps 1a and 1b. Examples of the amine include trimethylamine, triethylamine, tripropylamine, tributylamine, diisopropylethylamine, dimethylbenzylamine, N-methylmorpholine, tetramethylethylenediamine, and N,N'-dimethylpiperazine. The method can be referred to, for example, in WO2014/110329.

As in R¹, R^{1'} preferably represents a hydrocarbon group (the hydrocarbon group optionally contains a heteroatom between carbon atoms) optionally having one or more substituents, more preferably an alkyl group (the alkyl group optionally contains a heteroatom between carbon atoms) optionally having one or more substituents. The "one or more substituents" that can be contained in the hydrocarbon group and alkyl group preferably include a halo group, and even more preferably include a fluoro group. Specifically, R1' preferably represents a haloalkyl group (the haloalkyl group optionally contains a heteroatom between carbon atoms) optionally having one or more substituents, and more preferably a fluoroalkyl group (the fluoroalkyl group optionally contains a heteroatom between carbon atoms) optionally having one or more substituents. The fluoroalkyl group may be a perfluoroalkyl group. The "one or more substituents" that can be contained in the haloalkyl group and fluoroalkyl group preferably include a nitrile group, an ester group (e.g., R^{r}OCO-), a formyl group, and a sulfonyl-containing group (e.g., R^{r}OSO₂-). The "heteroatom" that can be included between carbon atoms of the hydrocarbon group, alkyl group, haloalkyl group, and fluoroalkyl group includes, but is not limited to, an oxygen atom, a sulfur atom, and a nitrogen atom. The number of carbon atoms in the hydrocarbon, alkyl group, haloalkyl group, and fluoroalkyl group is not limited as long as it is at least one. For example, it is in the range of 1 to 11, preferably in the range of 1 to 8, more preferably in the range of 1 to 5, and most preferably in the range 1 to 3.

The compounds represented by formula (1) can be used alone or in a combination of two or more.

### 2-2. Compound Represented by Formula (2a)

In formula (2a), R^{2a} and R^{3a} each preferably represent H or a C₁₋C₆ alkyl group, more preferably H or a C₁₋C₄ alkyl group, and even more preferably H or a C₁₋C₃ alkyl group. In one embodiment, it is preferred that (I) R^{2a} and R^{3a} are both H, (II) R^{2a} is H and R^{3a} is a C₁₋C₃ alkyl group, or (III) R^{2a} and R^{3a} are both C₁₋C₃ alkyl groups.

In formula (2a), R^{4a} is preferably a halogen or a hydrocarbon group optionally having one or more substituents, and more preferably a halogen or an alkyl group optionally having one or more substituents. The "substituent" that can be contained in the hydrocarbon group and the alkyl group preferably includes a halo group, and even more preferably includes a fluoro group.

R^{4a} is preferably a halogen or a haloalkyl group, more preferably F or a fluoroalkyl group, even more preferably F or a fluoro C₁₋C₆ alkyl group, still more preferably F or a fluoro C₁₋C₄ alkyl group, and particularly preferably F or a fluoro C₁₋C₃ alkyl group.

X^{1a} represents a halogen other than F; however, the reaction in step 1a can proceed. X^{1a} is preferably Cl, Br, or I, and more preferably Cl or Br.

The compounds represented by formula (2a) can be used alone or in a combination of two or more.

The amount of the compound represented by formula (2a) is not limited. The lower limit of the amount used is, for example, 0.5 mol or more, preferably 1 mol or more, more preferably 3 mol or more, and even more preferably 5 mol or more per mole of the compound represented by formula (1). The upper limit of the amount is, for example, 50 mol or less, preferably 40 mol or less, and even more preferably 30 mol or less per mole of the compound represented by formula (1). The lower limit and upper limit of the amount can be set to the range of any combination. When the compound represented by formula (2a) is used in excess, the unreacted compound represented by formula (2a) can be recovered for further use.

### 2-3. Halogenating Agent

The halogenating agent is not limited as long as it can introduce a halogen to the carbon atom replaced by R^{2a} and R^{3a} of the compound represented by formula (2a). Examples of the halogenating agent include compounds represented by formula (3a) or (3a').

In formula (3a), X² is preferably Cl, Br, or I.

In formula (3a), when Z is halogen, it may be the same as or different from X². The halogen is preferably Cl, Br, or I. In formula (3a), the halogenating agent wherein Z is halogen is, for example, I₂, Br₂, ICl, and BrI.

When Z is NZ¹Z², Z¹ and Z² each preferably represent an alkyl group, an alkylcarbonyl group, or an alkylsulfonyl group.

When Z is NZ¹Z², the halogenating agent is, for example, an N-haloamide compound. The N-haloamide compound is preferably a primary amide, a secondary amide, or a combination of these. Preferable examples of the primary amide or secondary amide include a carboxyamide, a sulfonamide, a lactam, a carbamate, an imide, an ureido, or a combination thereof. Examples of the amide include halogenated 5,5-dimethylhydantoin, 3-benzyl-5,5-dimethylhydantoin, 5-methyl-5-phenylhydantoin, 5,5-diphenylhydantoin, 5,5-hexamethylenehydantoin, 5,5-pentamethylenehydantoin, 5,5-tetramethylenehydantoin, succinimide, phthalimide, saccharin, isocyanuric acid, 5,5-dimethylbarbituric acid, glycoluril, 3a,6a-diphenylglycoluril, 3a,6a-dimethylglycoluril, 4,4,5,5-tetramethyl-2-imidazolidinone, and 4,4-dimethyl-2-oxazolidinone.

The primary amide is, for example, a carboxyamide. Such a primary carboxyamide is, for example, the following:
Z⁸C(=O)NX²Z⁹, such as a lactam or a peptide;
Z^{B}NX²C(=O)OZ⁹ (carbamate), such as t-BuX²NBoc and a 2-oxazolidinone derivative (e.g., 4,4-dimethyl-2-oxazolidinone);
Z^{B}NX²C(=O)NX²Z⁹ (urea derivative), such as a 2-imidazolidinone derivative (e.g., 4,4,5,5-tetramethyl-2-imidazolidinone), glycoluryl, and its derivatives (e.g., 3a,6a-diphenylglycol uryl and 3a,6a-dimethylglycol uryl).
Z⁸ and Z⁹ each independently represent an alkyl group optionally having one or more substituents, X² is as defined above, and two X²s present in the same molecule may be the same or different from each other.

The primary amide is, for example, a sulfonamide, phosphoramide, or nitramide. Examples of the sulfonamide include Z⁸S (=O) ₂NX²Z⁹ and Z⁸NX²S (=O)₂NX²Z⁹. Examples of the phosphoramide include Z⁸₂P(=O)NX²Z⁹. Examples of the nitramide include O₂N-NX²Z⁸ . Z⁸, Z⁹, and X² are as defined above.

The secondary amide is, for example, an imide ([C(=O)]₂NX²). Examples of the imide include succinimide and phthalimide. Examples of the secondary amide include Z⁸S (=O) ₂NX²C (=O) Z⁹, Z⁸S (=O) ₂NX²S (=O) ₂Z⁹, Z⁸X²NC (=O)NX²C (=O) Z⁹, Z⁸C (=O) NX²C (=O) NX²C (=O) Z⁹, Z⁸S (=O) ₂NX²C (=O)NX²C (=O)Z⁹, and Z⁸S (=O) ₂NX²C (=O) NX²S (=O) ₂Z⁹ (ureido) . Z⁸, Z⁹, and X² are as defined above. Non-limiting examples of ureido include a halogenated hydantoin derivative (e.g., 5,5-dimethylhydantoin, 3-benzyl-5,5-dimethylhydantoin, 5-methyl-5-phenylhydantoin, 5,5-diphenylhydantoin, 5,5-pentamethylenehydantoin, 5,5-hexamethylenehydantoin, 5,5-tetramethylenehydantoin), isocyanuric acid and a barbituric acid derivative (e.g., 5,5-diethylbarbituric acid, 5,5-dimethylbarbituric acid, 5-ethyl-5-isoamylbarbituric acid).

The amide is preferably a sulfonamide, lactam, carbamate, imide, or ureido. Preferable examples of the amide include halogenated 5-dimethylhydantoin, 3-benzyl-5,5-dimethylhydantoin, 5-methyl-5-phenylhydantoin, 5,5-diphenylhydantoin, 5,5-hexamethylenehydantoin, 5,5-pentamethylenehydantoin, 5,5-tetramethylenehydantoin, succinimide, phthalimide, saccharin, isocyanuric acid, 5,5-dimethylbarbituric acid, glycoluril, 3a,6a-diphenylglycoluril, 3a,6a-dimethylglycoluril, 4,4,5,5-tetramethyl-2-imidazolidinone, or 4,4-dimethyl-2-oxazolidinone.

The halogenating agent is, for example, a monohalogenated amide compound or a polyhalogenated amide compound.

Examples of the halogenating agent wherein Z is NZ¹Z² and Z¹ and Z² are bonded together to form a ring include 1,3-dichloro-5,5-dimethylhydantoin, 1,3-dibromo-5,5-dimethylhydantoin, 1,3-diiodo-5,5-dimethylhydantoin, N-chlorosuccinimide, N-bromosuccinimide, N-iodosuccinimide dichloroisocyanurate, dibromoisocyanurate, diiodoisocyanurate, N-chlorophthalimide, N-bromophthalimide, N-iodophthalimide, N-chlorosaccharin, N-bromosaccharin, N-iodosaccharin, 1-halo-5,5-dimethylhydantoin, 3-halo-5,5-dimethylhydantoin, 2,4,6,8-tetrahaloglycol-uryl, and mixtures thereof.

In formula (3a'), Q¹ to Q⁴ are not limited as long as they can form nitrogen-containing ions. Q¹ to Q⁴ each preferably represent H or a hydrocarbon group optionally having one or more substituents, more preferably H, an alkyl group optionally having one or more substituents, a cycloalkyl group optionally having one or more substituents, an aryl group optionally having one or more substituents, or an aralkyl group optionally having one or more substituents, even more preferably an alkyl group optionally having one or more substituents, and particularly preferably a C₁₋C₄ alkyl group optionally having one or more substituents. The "substituent" that can be contained in the hydrocarbon group, alkyl group, cycloalkyl group, aryl group, and aralkyl group includes, but is not limited to, a halo group, a hydroxyl group, and a mercapto group.

The ring formed by binding of any two of Q¹ to Q⁴ is, but is not limited to, a five- or six-membered ring, such as pyrrolidine, piperidine, and morpholine.

The halogenating agents can be used alone or in a combination of two or more.

The amount of the halogenating agent is not limited. The lower limit of the amount is, for example, 0.01 mol or more, preferably 0.05 mol or more, and even more preferably 0.1 mol or more per mole of the compound represented by formula (1). The upper limit of the amount is, for example, 10 mol or less, preferably 7 mol or less, and even more preferably 5 mol or less per mole of the compound represented by formula (1). The lower limit and the upper limit of the amount can be set to the range of any combination.

### 2-4. Catalyst

Step 1a can be performed in the absence of a catalyst, but it can be performed in the presence of a catalyst.

The catalyst is not limited as long as it catalyzes the reaction. Examples of the catalyst include a Lewis base and the like. Examples of the Lewis base include the compound represented by formula (3b).

In formula (3b), when L¹ is O, (S)ₘ, or (Se)ₖ, n is 2, each Z³ is preferably a hydrocarbon group optionally having one or more substituents, more preferably an alkyl group optionally having one or more substituents, a cycloalkyl group optionally having one or more substituents, an aryl group optionally having one or more substituents, or an aralkyl group optionally having one or more substituents; even more preferably an alkyl group optionally having one or more substituents, or an aryl group optionally having one or more substituents. The "substituent" that can be contained in the hydrocarbon group, alkyl group, cycloalkyl group, aryl group, and aralkyl group includes a halo group and an alkoxy group.

When L¹ represents N or P, n is 3 and Z³s each preferably represent a hydrocarbon group optionally having one or more substituents; more preferably an alkyl group optionally having one or more substituents, a cycloalkyl group optionally having one or more substituents, an aryl group optionally having one or more substituents, or an aralkyl group optionally having one or more substituents; even more preferably an alkyl group optionally having one or more substituents, or an aryl group optionally having one or more substituents.

Examples of the compound represented by formula (3b) include dialkyl ethers, such as diethyl ether; diaryl sulfides, such as diphenyl ether; dialkyl sulfides, such as dimethyl sulfide; diaryl sulfides, such as diphenyl sulfide; dialkyl disulfides, such as dimethyl disulfide; diaryldisulfides, such as diphenyl disulfide; dialkyl selenides, such as dimethyl selenide; diarylselenides, such as diphenyl selenide; dialkyldiselenides, such as dimethyldiselenide; diaryldiselenides, such as diphenyldiselenide; trialkylamines, such as triethylamine; triarylamines, such as triphenylamine; trialkylphosphines, such as triethylphosphine; triarylphosphines, such as triphenylphosphine, etc.; however, the examples are not limited thereto.

In formula (3c), Z⁴ is, for example, P(Z⁵)₃ (wherein Z⁵s each independently represent an organic group, and any two Z⁵s are optionally bound together to form a ring), C(Z⁶)₂ (wherein Z⁶s each independently represent an organic group, and any two Z⁶s are optionally bound together to form a ring), etc.

Z⁵s each preferably represent a hydrocarbon group optionally having one or more substituents, more preferably an alkyl group optionally having one or more substituents, a cycloalkyl group optionally having one or more substituents, an aryl group optionally having one or more substituents, or an aralkyl group optionally having one or more substituents.

Z⁵s each preferably represent N(Z⁷) ₂, wherein Z⁷s each independently represent an organic group and two Z⁷s are optionally bound together to form a ring.

Z⁷s each preferably represent a hydrocarbon group optionally having one or more substituents; more preferably an alkyl group optionally having one or more substituents, a cycloalkyl group optionally having one or more substituents, an aryl group optionally having one or more substituents, or an aralkyl group optionally having one or more substituents; and even more preferably an alkyl group optionally having one or more substituents.

The catalysts can be used alone or in a combination of two or more.

The amount of the catalyst is not limited. The lower limit of the amount is, for example, 0.001 mol or more, preferably 0.005 mol or more, and even more preferably 0.01 mol or more per mole of the compound represented by formula (1). The upper limit of the amount is, for example, 30 mol or less, preferably 20 mol or less, and even more preferably 10 mol or less per mole of the compound represented by formula (1). The lower limit and the upper limit of the amount can be set to the range of any combination.

### 2-5. Solvent

Step 1a is preferably performed in the presence of a solvent. The solvent is not limited, and examples include the following solvents.
- A hydrocarbon solvent (e.g., a chain hydrocarbon, such as n-hexane; and an aromatic hydrocarbon, such as benzene, toluene, and p-xylene)
- A halogenated solvent, such as a fluorinated solvent and a chlorinated solvent (e.g., a haloalkane, such as dichloromethane, dichloroethane, and perfluorohexane; a haloarene, such as chlorobenzene; a haloalkylarene, such as trifluoro toluene and hexafluorometa-xylene; a haloether (e.g., fluoroethers, such as 3 M^{™}Novec^{™}7200 and 3M^{™}Novec^{™}7300 produced by 3M))
- A nitrile-based solvent (e.g., a chain nitrile, such as acetonitrile, propionitrile, and acrylonitrile; a cyclic nitrile, such as benzonitrile)
- An amide-based solvent (e.g., a carboxylic acid amide (e.g., a chain amide, such as formamide, N-methylformamide, and N,N-dimethylformamide; a cyclic amide, such as N-methylpyrrolidone), a phosphoric acid amide (e.g., hexamethylphosphamide))
- An ether-based solvent (e.g., a chain ether, such as diethyl ether, ethylene glycol dimethyl ether, diethylene glycol dimethyl ether, triethylene glycol dimethyl ether; and a cyclic ether, such as tetrahydrofuran and dioxane)
- An urea-based solvent (e.g., N,N-dimethylpropyleneurea)
- An ester-based solvent (e.g., acetic acid ester)
- A sulfoxide-based solvent (e.g., dimethyl sulfoxide)
- A nitro-based solvent (e.g., nitromethane and nitrobenzene)
- A ketone-based solvent (e.g., acetone and methyl ethyl ketone)
- A mixture of two or more of these solvents

### 2-6. Temperature

The temperature in step 1a is not limited as long as the reaction proceeds. The lower limit of the temperature is, for example, -20°C or more, preferably 0°C or more, and more preferably 10°C or more. The upper limit of the temperature is, for example, 100°C or less, preferably 80°C or less, and even more preferably 50°C or less. The lower limit and the upper limit of the temperature can be set to the range of any combination.

### 2-7. Time

The time of step 1a is not limited as long as the reaction proceeds. The lower limit of the time is, for example, 3 hours or more, preferably 6 hours or more, and even more preferably 10 hours or more. The upper limit of the time is, for example, 72 hours or less and preferably 50 hours or less. The lower and upper limit of the time can be set to the range of any combination.

The reaction product obtained in step 1a may be purified by a conventional method, such as filtration, distillation, extraction, and column chromatography.

### 3. Method for Producing Compound Represented by Formula (4b)

In one embodiment, the method for producing the compound represented by formula (4b) includes step 1b of allowing the compound represented by formula (1) to react with the compound represented by formula (2b) in the presence of a halogenating agent and a catalyst.

### 3-1. Compound Represented by Formula (1)

The compound represented by formula (1) is as described in 2-1 above.

### 3-2. Compound Represented by Formula (2b)

In formula (2b), R^{2b} and R^{3b} each preferably represent H, a halogen, or an alkyl group optionally having a halo group as a substituent; more preferably H, a halogen, an alkyl group, or a fluoroalkyl group; even more preferably H, F, a C₁₋C₆ alkyl group, or a fluoro C₁₋C₆ alkyl group; still more preferably H, F, a C₁₋C₄ alkyl group, or a fluoro C₁₋C₄ alkyl group; and particularly H, F, a C₁₋C₃ alkyl group, or a fluoro C₁₋C₃ alkyl group. In one embodiment, it is preferable that (I) R^{2b} and R^{3b} are both H, (II) R^{2b} is H and R^{3b} is a C₁₋C₃ alkyl group, or (III) R^{2b} and R^{3b} are both C₁₋C₃ alkyl groups.

In one embodiment, R^{4b} preferably represents an organic group. In other embodiments, R^{4b}, for example, represents H, a halogen, or an alkyl group optionally having one or more substituents, preferably a halogen, or an alkyl group optionally having a halo group as a substituent, more preferably a halogen or a fluoroalkyl group, even more preferably F or a fluoroalkyl group, still more preferably F or a fluoro C₁₋C₆ alkyl group, particularly preferably F or a fluoro C₁₋C₄ alkyl group, and particularly more preferably F or a fluoro C₁₋C₃ alkyl group.

X^{1b} may be F or a halogen other than F. In one embodiment, when R^{4b} represents F or a fluoroalkyl group, X^{1b} is preferably Cl, Br, or I. In one embodiment, X^{1b} is preferably Cl or Br.

### 3-3. Halogenating Agent

The halogenating agent and the amount are as described in section 2-3 above.

### 3-4. Catalyst

The catalyst and the amount are as described in section 2-4 above.

### 3-5. Solvent

Step 1b is preferably performed in the presence of a solvent. The solvent is as described in section 2-5 above.

### 3-6. Temperature

The temperature in step 1b is not limited as long as the reaction proceeds. The lower limit of the temperature is, for example, -20°C or more, preferably 0°C or more, and more preferably 10°C or more. The upper limit of the temperature is, for example, 100°C or less, preferably 70°C or less, and even more preferably 50°C or less. The lower limit and the upper limit of the temperature can be set to the range of any combination.

### 3-7. Time

The time of step 1b is not limited as long as the reaction proceeds. The lower limit of the time is, for example, 3 hours or more, preferably 6 hours or more, and even more preferably 10 hours or more. The upper limit of the time is, for example, 70 hours or less, and preferably 50 hours or less. The lower and upper limit of the time can be set to the range of any combination.

The reaction product obtained in step 1b may be purified by a conventional method, such as filtration, distillation, extraction, and column chromatography.

### 4. Compound Represented by Formula (4a)

The compound represented by formula (4a) is as described in section 2 above.

### 5. Composition Containing Compound Represented by Formula (4b) 5-1. Compound Represented by Formula (4b)

The compound represented by formula (4b) is as described in section 3 above, and may be, for example, the compound represented by formula (4a). The compound represented by formula (4a) is as described in section 2 above. The content of the compound represented by formula (4b) is not limited, and can be, for example, 50 mass% or more, 60 mass% or more, 70 mass% or more, 80 mass% or more, 90 mass% or more, and/or 99 mass% or less or 95 mass% or less based on 100 mass% of the composition.

### 5-2. Other Components

The composition is not limited as long as it contains the compound represented by formula (4b), and may further contain other components. Examples of other components include halide ions, water, the compound represented by formula (2b), a solvent, a catalyst, a carboxylic acid, a halogen adduct of the compound represented by formula (2b), the compound represented by formula (1'), a cation represented by Y⁺, a halogenating agent, and oxygen. Other components can be impurities etc. in the production method of the compound represented by formula (4a) or (4b) as described in section 2 or 3 above. The other components can be used alone or in a combination of two or more.

### 5-3. Composition Containing Compound Represented by Formula (4b) and Halide Ion

The halide ion is not limited, and examples include a fluoride ion, a chloride ion, a bromide ion, and an iodide ion. The halide ion is preferably a chloride ion, a bromide ion, or an iodide ion. The halide ion can be, for example, an impurity in the production method of the compound represented by formula (4a) or (4b) as described in section 2 or Section 3 above, specifically, derived from a halogenating agent.

The content of the halide ion is not limited. The upper limit of the content is, for example, 10 mass% or less, and preferably 6 mass% or less based on 100 mass% of the compound represented by formula (4b) (or 100 mass% of the composition). The lower limit of the content is, for example, above the detection limit, and is, for example, 3 x 10⁻⁸ mass% or more, preferably 3 x 10⁻⁶ mass% or more, and more preferably 3 x 10⁻³ mass% or more based on 100 mass% of the compound represented by formula (4b) (or 100 mass% of the composition.) The lower limit and the upper limit of the content can be set to the range of any combination. The content can be determined by ion chromatography or gas chromatography-mass spectrometry (GC-MS).

### 5-3'. Composition Containing Compound Represented by Formula (4b), Halide Ion, and Catalyst

The halide ion includes those listed in section 5-3 above. Examples of the catalyst include those listed in section 2-4 or section 3-4 above. The catalyst can be derived, for example, from a catalyst that can be used in the method for producing the compound represented by formula (4a) or (4b) as described in section 2 or 3 above.

The content of the halide ion is not limited. The upper limit of the content is, for example, 10 mass% or less and preferably 6 mass% or less based on 100 mass% of the compound represented by formula (4b) (or 100 mass% of the composition). The lower limit of the content is, for example, above the detection limit, and is, for example, 3 x 10⁻⁸ mass% or more, preferably 10⁻⁶ mass% or more, and more preferably 10⁻³ mass% or more based on 100 mass% of the compound represented by formula (4b) (or 100 mass% of the composition.) The lower limit and the upper limit of the content can be set to the range of any combination. The content can be determined by ion chromatography or gas chromatography-mass spectrometry (GC-MS).

The catalyst content is not limited. The upper limit of the content is, for example, 10 mass% or less, and preferably 5 mass% or less based on 100 mass% of the compound represented by formula (4b) (or 100 mass% of the composition). The lower limit of the content is, for example, above the detection limit, and is, for example, 10⁻¹¹ mass% or more, preferably 10⁻⁹ mass% or more, and more preferably 10⁻³ mass% or more based on 100 mass% of the compound represented by formula (4b) (or 100 mass% of the composition). The lower limit and the upper limit of the content can be set to the range of any combination. The content can be determined by inductively coupled plasma mass spectrometry (IPC-MS), ¹H-NMR, gas chromatography (GC), or gas chromatography-mass spectrometry (GC-MS).

### 5-4. Composition Containing Compound Represented by Formula (4b) and Water

The water content is not limited. The upper limit of the content is, for example, 1 mass% or less, and preferably 0.5 mass% or less based on 100 mass% of the compound represented by formula (4b) (or 100 mass% of the composition). The lower limit of the content is, for example, above the detection limit, and is, for example, 10⁻⁸ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by the Karl Fischer method or ¹H-NMR.

### 5-4'. Composition Containing Compound Represented by Formula (4b), Water, and Catalyst

Examples of the catalyst include those listed in Item 2-4 or 3-4 above. The catalyst can be derived, for example, from a catalyst that can be used in the method for producing the compound represented by formula (4a) or (4b) as described in section 2 or 3 above.

The water content is not limited. The upper limit of the content is, for example, 1 mass% or less, and preferably 0.5 mass% or less based on 100 mass% of the compound represented by formula (4b) (or 100 mass% of the composition). The lower limit of the content is, for example, above the detection limit, and is, for example, 10⁻⁸ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by the Karl Fischer method or ¹H-NMR.

The catalyst content is not limited. The upper limit of the content is, for example, 10 mass% or less, and preferably 5 mass% or less based on 100 mass% of the compound represented by formula (4b) (or 100 mass% of the composition). The lower limit of the content is, for example, above the detection limit, and is, for example, 10⁻¹¹ mass% or more, preferably 10⁻⁹ mass% or more, and more preferably 10⁻³ mass% or more based on 100 mass% of the compound represented by formula (4b) (or 100 mass% of the composition). The lower limit and the upper limit of the content can be set to the range of any combination. The content can be determined by inductively coupled plasma mass spectrometry (IPC-MS), NMR, gas chromatography (GC), or gas chromatography-mass spectrometry (GC-MS).

### 5-5. Composition Containing Compound Represented by Formula (4b) and Compound Represented by Formula (2b)

The compound represented by formula (2b) is as described in section 3-2 above, and may be, for example, the compound represented by formula (2a). The compound represented by formula (2a) is as described in section 2-2 above. The compound represented by formula (2b) can be, for example, an unreacted raw material in the method for producing the compound represented by formula (4a) or (4b) as described in section 2 or 3 above.

The content of the compound represented by formula (2b) is not limited. The upper limit of the content is, for example, 10 mass% or less, and preferably 5 mass% or less based on 100 mass% of the compound represented by formula (4b) (or 100 mass% of the composition). The lower limit of the content is above the detection limit, and is, for example, 10⁻⁹ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by ¹H-NMR, gas chromatography (GC), or gas chromatography-mass spectrometry (GC-MS).

### 5-6. Composition Containing Compound Represented by Formula (4b) and Solvent

The solvent includes those listed in section 2-5 or 3-5 above. The solvent can be, for example, derived from a solvent that can be used in the method for producing the compound represented by formula (4a) or (4b) as described in section 2 or 3 above. The solvent can be optionally added after the production of the compound represented by formula (4b).

The content of the solvent is not limited. The upper limit of the content is, for example, 10 mass% or less, and preferably 5 mass% or less based on 100 mass% of the compound represented by formula (4b) (or 100 mass% of the composition). The lower limit of the content is above the detection limit, and is, for example, 10⁻⁹ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by ¹H-NMR, gas chromatography (GC), or gas chromatography-mass spectrometry (GC-MS) .

### 5-7. Composition Containing Compound Represented by Formula (4b) and Catalyst

Examples of the catalyst include those listed in section 2-4 or 3-4 above. The catalyst can be derived, for example, from a catalyst that can be used in the method for producing the compound represented by formula (4a) or (4b) as described in section 2 or 3 above.

The catalyst content is not limited. The upper limit of the content is, for example, 10 mass% or less, and preferably 5 mass% or less based on 100 mass% of the compound represented by formula (4b) (or 100 mass% of the composition). The lower limit of the content is, for example, above the detection limit, and is, for example, 10⁻¹¹ mass% or more, preferably 10⁻⁹ mass% or more, and more preferably 10⁻³ mass% or more based on 100 mass% of the compound represented by formula (4b) (or 100 mass% of the composition). The lower limit and the upper limit of the content can be set to the range of any combination. The content can be determined by inductively coupled plasma mass spectrometry (IPC-MS), ¹H-NMR, gas chromatography (GC), or gas chromatography-mass spectrometry (GC-MS).

### 5-8. Composition Containing Compound Represented by Formula (4b) and Carboxylic Acid

The carboxylic acid is not limited, and includes the compound represented by formula (6):

R¹-COOH (6)

wherein R¹ is as defined above. The carboxylic acid can be, for example, a hydrolysis product of the compound represented by formula (1') when the compound represented by formula (1) is generated from the compound represented by formula (1') in the reaction system in the method for producing the compound represented by formula (4a) or (4b) described in section 2 or 3 above.

The carboxylic acid content is not limited. The upper limit of the content is, for example, 10 mass% or less, and preferably 6 mass% or less based on 100 mass% of the compound represented by formula (4b) (or 100 mass% of the composition). The lower limit of the content is above the detection limit, and is, for example, 10⁻⁹ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by ¹H-NMR, gas chromatography (GC), or gas chromatography-mass spectrometry (GC-MS) .

### 5-9. Composition Containing Compound Represented by Formula (4b) and Halogen Adduct of Compound Represented by Formula (2b)

The halogen adduct of the compound represented by formula (2b) is not limited, and includes the compound represented by formula (7): 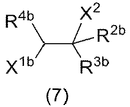 wherein R^{2b}, R^{3b}, R^{4b}, X^{1b}, and X² are as defined above.

The content of the halogen adduct is not limited. The upper limit of the content is, for example, 10 mass% or less, and preferably 6 mass% or less based on 100 mass% of the compound represented by formula (4b) (or 100 mass% of the composition). The lower limit of the content is above the detection limit, and is, for example, 3 x 10⁻⁹ mass% or more, and preferably 10⁻⁶ mass% or more based on 100 mass% of the compound represented by formula (4b) (or 100 mass% of the composition). The lower limit and the upper limit of the content can be set to the range of any combination. The content can be determined by ¹H-NMR, gas chromatography (GC), or gas chromatography-mass spectrometry (GC-MS) .

### 6. Method for Producing Compound Represented by Formula (5a)

The method for producing the compound represented by formula (5a) includes step 2a of allowing a reducing agent to the compound represented by formula (4a). The production method preferably includes step 1a.

### 6-1. Compound Represented by Formula (4a)

The compound represented by formula (4a) is as described in section 2 above.

### 6-2. Reducing Agent

The reducing agent is not limited as long as it can reduce the compound represented by formula (4a).

In one embodiment, the reducing agent is preferably an organometallic reagent. Examples of the organometallic reagent include Grignard reagents, including alkylmagnesium bromides, such as methylmagnesium bromide and ethylmagnesium bromide, arylmagnesium bromides, such as phenylmagnesium bromide, etc.

In one embodiment, the reducing agent is preferably at least one member selected from the group consisting of
(i) transition metals, alkali metals, and alkaline earth metals,
(ii) metal pairs of transition metals, and
(iii) mixtures of transition metals and acids or metal salts.

Examples of the transition metal in the reducing agent (i) include Zn, Cu, Fe, Mn, etc. Examples of the alkali metal in the reducing agent (i) include Na. Examples of the alkali earth metal in the reducing agent (i) include Mg.

The transition metal pairs in the reducing agent (ii) can include transition metals listed in the reducing agent (i), and examples include Zn/Cu etc.

Examples of the mixture of the reducing agent (iii) can contain the transition metal listed in the reducing agent (i). Examples include Zn/acetic acid, Zn/ZnCl₂, etc.

In one embodiment, the reducing agent is preferably a phosphorus-containing compound. Examples of the phosphorus-containing compound include triarylphosphines, such as triphenylphosphine; and compounds with multiple phosphorus atoms linked via a linker (e.g., alkylene), such as 1,2-bis(diphenylphosphino)ethane; trialkylphosphines, such as tributylphosphine. Phosphines substituted with a mixture of alkyl and aryl groups may be also used. In addition, examples of the phosphorus-containing compound include trialkyl phosphites, such as triethyl phosphite; and tri(mono or dialkylamino)phosphines, such as tri(diethylamino)phosphine.

The amount of the reducing agent is not limited. The lower limit of the amount is, for example, 0.01 mol or more, preferably 0.1 mol or more, and even more preferably 0.5 mol or more per mole of the compound represented by formula (4a). The upper limit of the amount is, for example, 10 mol or less, preferably 7 mol or less, and even more preferably 5 mol or less per mole of the compound represented by formula (4a). The lower limit and the upper limit of the amount can be set to the range of any combination.

### 6-3. Solvent

Step 2a is preferably performed in the presence of a solvent. The solvent is not limited, and examples include the following solvents.
- A hydrocarbon solvent (e.g., a chain hydrocarbon, such as n-hexane; and an aromatic hydrocarbon, such as benzene, toluene, and p-xylene)
- A halogenated solvent, such as a fluorinated solvent and a chlorinated solvent (e.g., a haloalkane, such as dichloromethane, dichloroethane, and perfluorohexane; a haloarene, such as chlorobenzene; a haloalkylarene, such as trifluoro toluene and hexafluorometa-xylene; a haloether (e.g., fluoroethers, such as 3 M^{™}Novec^{™}7200 and 3M^{™}Novec^{™}7300 produced by 3M))
- A nitrile-based solvent (e.g., a chain nitrile, such as acetonitrile, propionitrile, and acrylonitrile; a cyclic nitrile, such as benzonitrile)
- An amide-based solvent (e.g., a carboxylic acid amide (e.g., a chain amide, such as formamide, N-methylformamide, and N,N-dimethylformamide; a cyclic amide, such as N-methylpyrrolidone), a phosphoric acid amide (e.g., hexamethylphosphamide))
- An ether-based solvent (e.g., a chain ether, such as diethyl ether, ethylene glycol dimethyl ether, diethylene glycol dimethyl ether, triethylene glycol dimethyl ether; and a cyclic ether, such as tetrahydrofuran and dioxane)
- An urea-based solvent (e.g., N,N-dimethylpropyleneurea)
- An ester-based solvent (e.g., acetic acid ester)
- A sulfoxide-based solvent (e.g., dimethyl sulfoxide)
- A nitro-based solvent (e.g., nitromethane and nitrobenzene)
- A ketone-based solvent (e.g., acetone and methyl ethyl ketone)
- Alcohol-based solvent (e.g., methanol and ethanol)
- Acid (e.g., acetic acid)
- A mixture of these two or more solvents

### 6-4. Temperature

The temperature in step 2a is not limited as long as the reaction proceeds. The lower limit of the temperature is, for example, -70°C or more, preferably -50°C or more, and more preferably -30°C or more. The upper limit of the temperature is, for example, 200°C or less, preferably 150°C or less, and even more preferably 100°C or less. The lower limit and the upper limit of the temperature can be set to the range of any combination.

### 6-5. Time

The time of step 2a is not limited as long as the reaction proceeds. The lower limit of the time varies depending on the type of the reducing agent, and is, for example, 5 minutes or more, and preferably 10 minutes or more. The upper limit of the time is, for example, 12 hours or less and preferably 6 hours or less. The lower and upper limit of the time can be set to the range of any combination.

The reaction product obtained in step 2a may be purified by a conventional method, such as filtration, distillation, extraction, and column chromatography.

### 7. Method for Producing Compound Represented by Formula (5b)

The method for producing the compound represented by formula (5b) includes step 1b, and step 2b of allowing the compound represented by formula (4b) to react with a reducing agent.

### 7-1. Step 1b

Step 1b is as described in section 3 above.

### 7-2. Compound Represented by Formula (4b)

The compound represented by formula (4b) is as described in section 3 above.

### 7-3. Reducing Agent

The reducing agent and the amount thereof are as described in section 6-2 above.

### 7-4. Solvent

Step 2b is preferably performed in the presence of a solvent. The solvent is as described in section 6-3 above.

### 7-5. Temperature and Time

The temperature and time for step 2b can be selected from the same range as the temperature and time for step 2a, described in section 6-4 and section 6-5 above, respectively.

The reaction product obtained in step 2b may be purified by a conventional method, such as filtration, distillation, extraction, and column chromatography.

### 8. Compound Represented by Formula (5c)

In formula (5c), when R^{1c} is a fluoroalkyl group having 2 or more carbon atoms or a fluoroalkoxy group having 1 or more carbon atoms, each optionally having one or more substituents, examples of the substituent include a nitrile group, an ester group (e.g., R^{r}OCO-), a formyl group, a sulfonyl-containing group (e.g., R^{r}OSO₂-), etc. The fluoroalkyl group or fluoroalkoxy group may be a perfluoroalkyl group or a perfluoroalkoxy group. The carbon number of the fluoroalkyl group or fluoroalkoxy group is, for example, 11 or less, preferably 10 or less, and more preferably 9 or less. The "heteroatom" that can be contained between carbon atoms of the fluoroalkyl group or the fluoroalkoxy group includes, but is not limited to, an oxygen atom, a sulfur atom, and a nitrogen atom.

Examples of R^{1c} include
CF₃-O-
CF₃-CF₂-,
CF₃-CF₂-CF₂-,
CF₃-CF₂-CF(CF₃)-,
CF₃-O-CF₂-,
CF₃-CF₂-O-,
CF₃-CF₂-CF₂-CF₂-,
CF₃-CF₂-O-CF₂-,
CF₃-O-CF₂-O-,
CF₃-CF₂-CF₂-CF₂-CF₂-,
CF₃-CF₂-CF₂-O-CH₂-,
CF₃-CF₂-CF₂-O-CF₂-,
CF₃-CF₂-CF₂-CF₂-CF₂-CF₂-,
CF₃-CF₂-CF₂-CF₂-CF₂-CF₂-CF₂-,
CF₃-CF(CF₃)-,
CF₃-CF₂-CF₂-O-CF(CF₃)-
CF₃-CF₂-CF₂-O-CF(CF₃)-CF₂-O-CF(CF₃)-,
CF₃CF₂-CF₂-O-[CF(CF₃)-CF₂-O-]₂-CF(CF₃)-, and like fluoro C₂₋₁₁ alkyl groups, fluoro C₁₋C₃ alkoxy groups, fluoro C₁₋C₃ alkoxyfluoro C₁₋C₃ alkoxy groups, fluoro C₁₋C₃ alkoxyfluoro C₁₋C₃ alkoxyfluoro C₁₋C₃ alkoxy groups, and the like.

In formula (5c), R^{2c} and R^{3c} each preferably represent H or a C₁₋C₆ alkyl group, more preferably H or a C₁₋C₄ alkyl group, and even more preferably H or a C₁₋C₃ alkyl group. In one embodiment, it is preferred that (I) R^{2c} and R^{3c} are both H, (II) R^{2c} is H and R^{3c} is a C₁₋C₃ alkyl group, or (III) R^{2c} and R^{3c} are both C₁₋C₃ alkyl groups.

### 9. Composition Containing Compound Represented by Formula (5b) 9-1. Compound Represented by Formula (5b)

The compound represented by formula (5b) is as described in section 7 above, and may be, for example, the compound represented by formula (5a) or (5c). The compounds represented by formulas (5a) and (5c) are as described in sections 6 and 8 above, respectively. The content of the compound represented by formula (5b) is not particularly limited, but can be, for example, 50 mass% or more, 60 mass% or more, 70 mass% or more, 80 mass% or more, or 90 mass% or more, and/or 99 mass% or less or 95 mass% or less based on 100 mass% of the composition.

### 9-2. Other Components

The composition is not limited as long as it contains the compound represented by formula (5b), and may further contain other components. Examples of other components include halide ions, water, the compound represented by formula (2b), a solvent, a catalyst, a carboxylic acid, a halogen adduct of the compound represented by formula (2b), the compound represented by formula (4b), a reducing agent, and oxygen. Other components can be impurities etc. in the production method of the compound represented by formula (5a) or (5b) as described in section 6 or 7 above. The other components can be used alone or in a combination of two or more.

### 9-3. Composition Comprising Compound Represented by Formula (5b) and Halide Ion

The halide ion is not limited, and examples include a fluoride ion, a chloride ion, a bromide ion, and an iodide ion. The halide ion is preferably a chloride ion, a bromide ion, or an iodide ion. The halide ion can be, for example, an impurity in the production method of the compound represented by formula (5a) or (5b) as described in section 6 or 7 above, specifically, derived from a halogenating agent.

The content of the halide ion is not limited. The upper limit of the content is, for example, 15 mass% or less and preferably 10 mass% or less based on 100 mass% of the compound represented by formula (5b) (or 100 mass% of the composition). The lower limit of the content is, for example, above the detection limit, and is, for example, 3 x 10⁻⁸ mass% or more, preferably 10⁻⁶ mass% or more, more preferably 10⁻³ mass% or more based on 100 mass% of the compound represented by formula (5b) (or 100 mass% of the composition.) The lower limit and the upper limit of the content can be set to the range of any combination. The content can be determined by ion chromatography or gas chromatography-mass spectrometry (GC-MS).

### 9-4. Composition Containing Compound Represented by Formula (5b) and Water

The water content is not limited. The upper limit of the content is, for example, 2 mass% or less, and preferably 1 mass% or less based on 100 mass% of the compound represented by formula (5b) (or 100 mass% of the composition). The lower limit of the water content is, for example, above the detection limit, and is, for example, 10⁻⁸ mass% or more, and preferably 10⁻⁶ mass% or more based on 100 mass% of the compound represented by formula (5b) (or 100 mass% of the composition). The lower limit and the upper limit of the content can be set to the range of any combination. The content can be determined by the Karl Fischer method or ¹H-NMR.

### 9-5. Composition Containing Compound Represented by Formula (5b) and Compound Represented by Formula (2b)

The compound represented by formula (2b) is as described in section 3-2 above, and may be, for example, the compound represented by formula (2a). The compound represented by formula (2a) is as described in section 2-2 above. The compound represented by formula (2b) is, for example, derived from the raw material of step 1a or 1b described in section 6 or 7.

The content of the compound represented by formula (2b) is not limited. The upper limit of the content is, for example, 10 mass% or less, and preferably 6 mass% or less based on 100 mass% of the compound represented by formula (5b) (or 100 mass% of the composition). The lower limit of water content is, for example, above the detection limit, and is, for example, 10⁻⁹ mass% or more, and preferably 10⁻⁶ mass% or more based on 100 mass% of the compound represented by formula (5b) (or 100 mass% of the composition). The lower limit and the upper limit of the content can be set to the range of any combination. The content can be determined by ¹H-NMR, gas chromatography (GC), or gas chromatography-mass spectrometry (GC-MS).

### 9-6. Composition Containing Compound Represented by Formula (5b) and Solvent

Examples of the solvent include those described in section 2-5 or 3-5 above. The solvent can be, for example, derived from a solvent that can be used in the method for producing the compound represented by formula (5a) or (5b) as described in section 6 or 7 above. The solvent can be, for example, one that can be optionally added after the production of the compound represented by formula (5b).

The content of the solvent is not particularly limited. The upper limit of the content is, for example, 10 mass% or less, and preferably 6 mass% or less based on 100 mass% of the compound represented by formula (5b) (or 100 mass% of the composition). The lower limit of the content is, for example, above the detection limit, and is, for example, 10⁻⁹ mass% or more, and preferably 10⁻⁶ mass% or more based on 100 mass% of the compound represented by formula (5b) (or 100 mass% of the composition). The lower limit and the upper limit of the content can be set to the range of any combination. The content can be determined by 1H-NMR, gas chromatography (GC), or gas chromatography-mass spectrometry (GC-MS).

### 9-7. Composition Containing Compound Represented by Formula (5b) and Carboxylic Acid

The carboxylic acid is not limited, and examples include the compound represented by formula (6) etc. The carboxylic acid can be, for example, a hydrolysis product of the compound represented by formula (1') when the compound represented by formula (1) is generated from the compound represented by formula (1') in the reaction system in step 1a or 1b described in section 6 or 7 above.

The carboxylic acid content is not limited. The upper limit of the content is, for example, 10 mass% or less, and preferably 6 mass% or less based on 100 mass% of the compound represented by formula (5b) (or 100 mass% of the composition). The lower limit of the content is, for example, above the detection limit, and is, for example, 10⁻⁹ mass% or more, and preferably 10⁻⁶ mass% or more based on 100 mass% of the compound represented by formula (5b) (or 100 mass% of the composition). The lower limit and the upper limit of the content can be set to the range of any combination. The content can be determined by ¹H-NMR, gas chromatography (GC), or gas chromatography-mass spectrometry (GC-MS).

### 9-8. Composition Containing Compound Represented by Formula (5b) and Compound Represented by Formula (4b)

The compound represented by formula (4b) is as described in section 3 above, and may be, for example, the compound represented by formula (4a). The compound represented by formula (4a) is as described in section 2 above. The compound represented by formula (4b) can be, for example, an unreacted raw material in step 2a or 2b as described in section 6 or 7 above.

The content of the compound represented by formula (4b) is not limited. The upper limit of the content is, for example, 10 mass% or less, and preferably 5 mass% or less based on 100 mass% of the compound represented by formula (5b) (or 100 mass% of the composition). The lower limit of the content is, for example, above the detection limit, and is, for example, 10⁻⁶ mass% or more, and preferably 10⁻⁶ mass% or more based on 100 mass% of the compound represented by formula (5b) (or 100 mass% of the composition). The lower limit and the upper limit of the content can be set to the range of any combination. The content can be determined by ¹H-NMR, gas chromatography (GC), or gas chromatography-mass spectrometry (GC-MS).

### Examples

One embodiment of the present disclosure is described in more detail below with reference to Examples; however, the present disclosure is not limited thereto.

### Example 1 Synthesis of 1-(1-chloro-1-fluoro-2-iodoethoxy)perfluorohexane

58.1 mg of potassium fluoride (fluorinating agent) and 1 ml of triglyme were added to a 20-mL pressure-resistant container under nitrogen atmosphere. 316 mg of perfluorohexanoyl fluoride and 0.25 ml of triglyme were added to the container. 162 mg of iodine monochloride (halogenating agent) and 0.25 ml of triglyme were added to the container. The container was cooled to 0°C or less, and 1.59 g of 1-chloro-1-fluoroethane was added. The container was stirred at 30°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 21%. ¹⁹F NMR (376 MHz, CDCl₃): δ -59.9 to -60.0 (m, 1F), -80.6 (t, 3F), -82.0 to -85.7 (m, 2F), -121.8 (s, 2F), -122.7 (s, 2F), 125.3 (s, 2F), -125.9 to -126.0 (t, 2F), ¹H NMR (400 MHz, CDCl₃): δ 3.94 to 3.88 (m, 2H).

The same fluoroether as that of Example 1 was synthesized by operating in the same manner as in Example 1, except that the types of fluorinating agent and halogenating agent were changed to the compounds shown in Table 1.

**Table 1**

| | Fluorinating agent | Halogenating agent | Yield (%) ¹⁹F NMR |
|---|---|---|---|
| Example 1 | Potassium fluoride | Iodine monochloride | 21 |
| Example 2 | Potassium fluoride | Iodine monochloride | 18 |
| Example 3 | Potassium fluoride | Iodine | 12 |
| Example 4 | Potassium fluoride | 1,3-Diiodo-5,5-dimethylhydantoin | 2 |
| Example 5 | Cesium fluoride | Iodine monochloride | 17 |
| Example 6 | Tetramethylammonium fluoride | Iodine | 10 |
| Example 7 | Tetramethylammonium fluoride | N-iodosaccharin | 6 |
| Example 8 | Tetramethylammonium fluoride | Iodine monochloride | 9 |

### Example 9 Reaction extension in synthesis of 1-(1-chloro-1-fluoro-2-iodoethoxy)perfluorohexane

After the reaction of Example 1 was performed, 162 mg of iodine monochloride was further added to the container. The container was cooled again to 0°C or less, and 1.62 g of 1-chloro-1-fluoroethane was added. The container was stirred at 30°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 39%.

### Example 10 Synthesis of 1-(2-bromo-1-chloro-1-fluoroethoxy)perfluorohexane

69.7 mg of potassium fluoride and 1 mL of triglyme were added to a 20-mL pressure-resistant container under nitrogen atmosphere. 316 mg of perfluorohexanoyl fluoride and 1 ml of triglyme were added to the container. 1,3-Dibromo-5,5-dimethylhydantoin (DBDMH) (286 mg) and triphenylphosphine sulfide (catalyst) (29.4 mg) were added to the container. The container was cooled to 0°C or less, and 1.60 g of 1-chloro-1-fluoroethane was added. The container was stirred at 40°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 31%. The residual ratio of unreacted perfluorohexanoyl fluoride was 57%. The conversion yield in consideration of the unreacted acid fluoride was 72%.

¹⁹F NMR (376 MHz, CDCl₃): is -65.6 to -65.7 (m, 1F), -82.4 (t, 3F), -82.8 to -83.2 (m, 1F), -86.0 to -86.4 (m, 1F), -123.3 (s, 2F), - 124.1 (s, 2F), 126.8 (s, 2F), -127.5 (s, 2F),
¹H NMR (400 MHz, CDCl₃): δ 3.95 to 3.91 (m, 2H).

The same fluoroether as that of Example 10 was synthesized by operating in the same manner as in Example 10, except that the catalyst was changed as shown in Table 2.

**Table 2**

| | Catalyst | Yield (%) ¹⁹F NMR |
|---|---|---|
| Example 11 | Triphenylphosphine sulfide | 31 |
| Example 12 | 1,3-Dihydro-4,5-dimetyl-1,3-bis(1-metyletyl)-2H-imidazole-2-thione | 26 |
| Example 13 | Triphenylphosphine oxide | 22 |
| Example 14 | Hexamethylphosphoramide | 23 |
| Example 15 | None | 17 |

### Example 16 Synthesis of 1-(2-bromo-1-chloro-1-fluoroethoxy)perfluorohexane using bromine

69.7 mg of potassium fluoride and 1 mL of triglyme were added to a 20-mL pressure-resistant container under nitrogen atmosphere. 316 mg of perfluorohexanoyl fluoride and 1 ml of triglyme were added to the container. Bromine (156 mg) was added to the container. The container was cooled to 0°C or less, and 1.60 g of 1-chloro-1-fluoroethane was added. The container was stirred at 40°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 43%. The obtained crude product was purified by column chromatography, thereby obtaining the title fluoroether with a yield of 40%.

The halide ion concentrations of the obtained fluoroether were measured by ion chromatography. As a result, the fluoride ion concentration was 2.1×10⁻³ mass%, the chloride ion concentration was 5.3×10⁻³ mass%, the bromide ion concentration was 0.4 mass%, and the iodide ion concentration was 3.7×10⁻³ mass%. Further, the water concentration measured with a Karl Fischer moisture meter was 8.5×10⁻² mass%.

### Example 17 Synthesis of 1-((1-fluorovinyl)oxy)perfluorohexane from 1-(1-chloro-1-fluoro-2-iodoethoxy)perfluorohexane

54.2 mg of 1-(1-chloro-1-fluoro-2-iodoethoxy)perfluorohexane (substrate) was added to a 10-mL glass container, and the inside of the container was replaced with nitrogen. 0.25 ml of diglyme (solvent) was added to the container. 35.9 mg of trisdiethylaminophosphine (reducing agent) was added to the container. The resulting mixture was stirred at -20°C for 1 hour. As a result of ¹⁹F NMR analysis, the title fluoroether was produced with a yield of over 99%.

¹⁹F NMR (376 MHz, CDCl₃): δ -81.7 to -81.9 (dt, 1F), -82.9 to - 82.9 (t, 3F), -86.9 (br, 2F), -124.5 (s, 2F), -125.0 (s, 2F), 127.4 to 127.5 (t, 2F), -128.2 to -128.3 (t, 2F),

¹H NMR (400 MHz, CDCl₃): δ 4.47 to 4.28 (m, 2H).

The same vinyl ether as that of Example 17 was synthesized by operating in the same manner as in Example 17, except that the reducing agent, solvent, substrate concentration, reaction temperature, and time were changed as shown in Table 3.

**Table 3**

| | Reducing agent | Solvent | Substrate concentration (M) | Reaction temperature (°C) | Time (h) | Yield (%) ¹⁹F NMR |
|---|---|---|---|---|---|---|
| Example 18 | Trisdiethylaminophosphine | Diglyme | 1.0 | -20 | 1 | >99 |
| Example 19 | Trisdiethylaminophosphine | Benzonitrile | 1.0 | -10 | 3 | 94 |
| Example 20 | Trisdiethylaminophosphine | Benzonitrile | 1.0 | -10 | 4 | 90 |
| Example 21 | Tris-tert-butylphosphine | Acetonitrile | 1.0 | -20 | 1 | 93 |
| Example 22 | Triphenylphosphine | Benzonitrile | 1.0 | r.t. | 18 | 18 |
| Example 23 | Ethylmagnesium bromide | Diglyme | 0.4 | 0 | 3 | 61 |
| Example 24 | Phenylmagnesium bromide | Diglyme | 0.4 | 0 | 4 | 69 |
| Example 25 | Zinc | Dimethoxyethane | 0.4 | 80 | 4 | 38 |
| Example 26 | Zinc/zinc chloride | Ethanol | 0.4 | 60 | 4 | 44 |

### Example 27 Synthesis of 1-((1-fluorovinyl)oxy)perfluorohexane from (2-bromo-1-chloro-1-fluoroethoxy)perfluorohexane

75.8 mg of (2-bromo-1-chloro-1-fluoroethoxy)perfluorohexane was added to a 10-mL glass container, and the inside of the container was replaced with nitrogen. 0.15 mL of acetonitrile was added to the container. 49.9 mg of trisdiethylaminophosphine was added to the container. The resulting mixture was stirred at -20°C for 3 hours. As a result of ¹⁹F NMR analysis, the title fluoroether was produced with a yield of 61%.

### Example 28 Synthesis of 1-(2-(1-chloro-1-fluoro-2-iodoethoxy)perfluoropropoxy)perfluoropropane

58.1 mg of potassium fluoride and 1 ml of triglyme were added to a 20-mL pressure-resistant container under nitrogen atmosphere. 332 mg of 2-(perfluoropropoxy)perfluoropropanoyl fluoride and 0.25 ml of triglyme were added to the container. 162 mg of iodine monochloride and 0.25 ml of triglyme were added to the container. The container was cooled to 0°C or less, and 1.26 g of 1-chloro-1-fluoroethane was added. The container was stirred at 30°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 22%.

¹⁹F NMR (376 MHz, CDCl₃): δ -59.4 to -59.7 (m, 1F), -79.4 to -79.5 (t, 3F), -80.4 to -80.5 (m, 2F), -81.0 (m, 3F), -85.4 to -86.4 (dd, 1F), -129.3 (s, 2F), -145.2 (s, 1F).
¹H NMR (400 MHz, CDCl₃): δ 3.92 to 3.85 (2H)

Perfluorohexane and methanol were added to the crude product, and liquid separation was performed. As a result of ¹⁹F NMR analysis, the title fluoroether was obtained with a yield of 19%.

The water concentration measured by ¹H NMR was 5.7 mass%, and the concentration of triglyme was 2.4 mass%.

### Example 29 Synthesis of 1-(2-((1-fluorovinyl)oxy)perfluoropropoxy)perfluoropropane

42.8 mg of 1-(2-(1-chloro-1-fluoro-2-iodoethoxy)perfluoropropoxy)perfluoropropane was added to a 10-ml glass container, and the inside of the container was replaced with nitrogen. 0.08 ml of acetonitrile was added to the container. 27.5 mg of trisdiethylaminophosphine was added to the container. The resulting mixture was stirred at -20°C for 1 hour. As a result of ¹⁹F NMR analysis, the title fluoroether was produced with a yield of 65%.
¹⁹F NMR (376 MHz, CDCl₃) : δ -83.80 (br)
¹H NMR (400 MHz, CDCl₃): δ 4.46 to 4.26 (2H)

### Example 30 Synthesis of 1-(1-chloro-1-fluoro-2-iodoethoxy)-3-(perfluoromethoxy)perfluoropropane

58.1 mg of potassium fluoride and 1 ml of triglyme were added to a 20-mL pressure-resistant container under nitrogen atmosphere. 232 mg of 3-(perfluoromethoxy)perfluoropropanoyl fluoride and 0.25 ml of triglyme were added to the container. 162 mg of iodine monochloride and 0.25 ml of triglyme were added to the container. The container was cooled to 0°C or less, and 0.98 g of 1-chloro-1-fluoroethane was added. The container was stirred at 30°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 36%.
¹⁹F NMR (376 MHz, CDCl₃): δ -59.4 to -59.5 (m, 1F)
¹H NMR (400 MHz, CDCl₃): δ 3.92 to 3.88 (2H).

The obtained fluoroether was analyzed by ¹H NMR. As a result, the obtained fluoroether contained 4.4 mass% of water and 3.6 mass% of triglyme.

### Example 31 Synthesis of 1-((1-fluorovinyl)oxy)-3-(perfluoromethoxy)perfluoropropane

35.8 mg of 1-(1-chloro-1-fluoro-2-iodoethoxy)-3-(perfluoromethoxy)perfluoropropane was added to a 10-mL glass container, and the inside of the container was replaced with nitrogen. 0.18 ml of acetonitrile was added to the container. 28 mg of trisdiethylaminophosphine was added to the container. The resulting mixture was stirred at -20°C for 1 hour. As a result of ¹⁹F NMR analysis, the title fluoroether was produced with a yield of 30%.
¹⁹F NMR (376 MHz, CDCl₃): δ -85.1 to -85.2 (dt, 1F)
¹H NMR (400 MHz, CDCl₃): δ 4.46 to 4.24 (2H).

### Example 32 Composition 1 of 1-((1-fluorovinyl)oxy)-3-(perfluoromethoxy)perfluoropropane

1 ml of triglyme was added to 29.6 mg of 1-((1-fluorovinyl)oxy)-3-(perfluoromethoxy)perfluoropropane obtained in Example 31 to prepare a triglyme solution containing 2.9 mass% of 1-((1-fluorovinyl)oxy)-3-(perfluoromethoxy)perfluoropropane. The solution was heated at 50°C for 2 hours. As a result of ¹⁹F NMR analysis, 1-((1-fluorovinyl)oxy)-3-(perfluoromethoxy)perfluoropropane was reduced by 2 mass%.

### Example 33 Composition 2 of 1-((1-fluorovinyl)oxy)-3-(perfluoromethoxy)perfluoropropane

0.5 ml of triglyme and 90 µl of water were added to 14.8 mg of 1-((1-fluorovinyl)oxy)-3-(perfluoromethoxy)perfluoropropane obtained in Example 31 to prepare a triglyme solution containing 2.5 mass% of 1-((1-fluorovinyl)oxy)-3-(perfluoromethoxy)perfluoropropane and 15 mass% of water. The solution was heated at 50°C for 2 hours. As a result of ¹⁹F NMR analysis, 1-((1-fluorovinyl)oxy)-3-(perfluoromethoxy)perfluoropropane was reduced by 23 mass%.

### Example 34 Composition 3 of 1-((1-fluorovinyl)oxy)-3-(perfluoromethoxy)perfluoropropane

0.5 ml of triglyme, 90 µl of water, and 29 mg of potassium fluoride were added to 14.8 mg of 1-((1-fluorovinyl)oxy)-3-(perfluoromethoxy)perfluoropropane obtained in Example 31 to prepare a triglyme solution containing 2.4 mass% of 1-((1-fluorovinyl)oxy)-3-(perfluoromethoxy)perfluoropropane, 14 mass% of water, and 5 mass% of potassium fluoride. As a result, the solution was separated. The solution was heated at 50°C for 2 hours. As a result of ¹⁹F NMR analysis, 1-((1-fluorovinyl)oxy)-3-(perfluoromethoxy)perfluoropropane was reduced by 9 mass%.

### Example 35 Composition 4 of 1-((1-fluorovinyl)oxy)-3-(perfluoromethoxy)perfluoropropane

1 ml of tetrahydrofuran was added to 29.6 mg of 1-((1-fluorovinyl)oxy)-3-(perfluoromethoxy)perfluoropropane obtained in Example 31 to prepare a tetrahydrofuran solution containing 3.2 mass% of 1-((1-fluorovinyl)oxy)-3-(perfluoromethoxy)perfluoropropane. The solution was heated at 50°C for 20 hours. As a result of ¹⁹F NMR analysis, 1-((1-fluorovinyl)oxy)-3-(perfluoromethoxy)perfluoropropane was reduced by 16 mass%.

### Example 36 Composition 5 of 1-((1-fluorovinyl)oxy)-3-(perfluoromethoxy)perfluoropropane

1 ml of a 1M tetrahydrofuran solution of tetra-n-butylammonium fluoride was added to 29.6 mg of 1-((1-fluorovinyl)oxy)-3-(perfluoromethoxy)perfluoropropane obtained in Example 31 to prepare a tetrahydrofuran solution containing 3.2 mass% of 1-((1-fluorovinyl)oxy)-3-(perfluoromethoxy)perfluoropropane and 28 mass% of tetra-n-butylammonium fluoride. The solution was heated at 50°C for 20 hours. As a result of ¹⁹F NMR analysis, 1-((1-fluorovinyl)oxy)-3-(perfluoromethoxy)perfluoropropane was reduced by 41 mass%.

### Example 35 Synthesis of 1-(1-bromo-1-fluoro-2-iodoethoxy)perfluorohexane

58.1 mg of potassium fluoride and 1 ml of triglyme were added to a 20-mL pressure-resistant container under nitrogen atmosphere. 316 mg of perfluorohexanoyl fluoride and 0.25 ml of triglyme were added to the container. 162 mg of iodine monochloride and 0.25 ml of triglyme were added to the container. The container was cooled to 0°C or less, and 1.48 g of 1-bromo-1-fluoroethane was added. The container was stirred at 30°C for 18 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 24%.

¹⁹F NMR (376 MHz, CDCl₃): δ -48.5 to -48.6 (m, 1F), -81.9 to -82.0 (m, 5F), -119.5 (t, 2F), -123.3 (s, 2F), 123.9 (s, 2F), -127.1 (t, 2F).

### Example 36 Synthesis of 1-(1-chloro-1-fluoro-2-iodoethoxy)perfluorooctane

186 mg of tetramethylammonium fluoride and 3.5 ml of triglyme were added to a 20-mL pressure-resistant container under nitrogen atmosphere. 832 mg of perfluorooctanoyl fluoride and 0.5 ml of triglyme were added to the container. 618 mg of N-iodosaccharin was added to the container. The container was cooled to 0°C or less, and 1.37 g of 1-chloro-1-fluoroethane was added. The container was stirred at 30°C for 24 hours. The obtained reaction mixture was added dropwise to a mixture of an aqueous potassium carbonate solution and an aqueous sodium sulfite solution, and extracted with ethyl acetate. As a result of ¹⁹F NMR analysis, the title fluoroether was produced with a yield of 21%.

¹⁹F NMR (376 MHz, CD₃COCD₃): δ -59.3 to -59.5 (m, 1F), -81.5 (t, J = 18.4 Hz, 3F), -82.4 to 82.5 (m, 1F), -85.8 to -86.2 (m, 1F), - 121.8 to -122.4 (m, 6F), -122.9 to -123.2 (m, 2F), -125.9 to - 126.0 (m, 2F), -126.5 to -126.7 (m, 2F).

¹H NMR (400 MHz, CD₃COCD₃): δ 4.35 (s, 1H), 4.31 (dd, J=12.8, 16.0 Hz, 1H).

### Example 37 Synthesis of 1-((1-fluorovinyl)oxy)perfluorooctane

64 mg of 1-(1-chloro-1-fluoro-2-iodoethoxy)perfluorooctane was added to a 10-mL glass container, and the inside of the container was replaced with nitrogen. 0.25 ml of diglyme was added to the container. 35 µL of a 3M phenylmagnesium bromide ether solution was added to the container. The resulting mixture was stirred at 50°C for 3 hours, and added dropwise to 1M HCl, followed by extraction with ether. As a result of ¹⁹F NMR analysis, the title fluoroether was produced with a yield of 55%. After the solvent was distilled off, the title vinyl ether was obtained with a yield of 35%.

¹⁹F NMR (376 MHz, CD₃COCD₃): δ -81.5 to -81.6 (m, 1F), -81.9 (t, J=12.0 Hz, 3F), -85.6 (t, J=9.8 Hz, 2F), -122.4 to -122.5 (m, 6F), -123.3 to -123.5 (m, 2F), -126.0 to -126.1 (m, 2F), -126.8 to -127.0 (m, 2F).

¹H NMR (400 MHz, CDCl₃): δ 4.47 (dd, J=5.2, 6.4 Hz, 1H), 4.37 (dd, J=4.4, 5.6 Hz, 1H).

The halide ion concentrations of the obtained vinyl ether were measured by ion chromatography. As a result, the chloride ion concentration was 8.7×10⁻³ mass%, and the fluoride ion concentration and the iodide ion concentration were equal to or below the detection limits. Further, the water concentration measured with a Karl Fischer moisture meter was 8.5×10⁻² mass%.

### List of Embodiments

### [Embodiment 1]

A method for producing a compound represented by formula (4a): 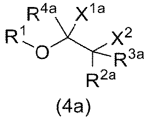 wherein
R¹ represents an organic group,
R^{2a} and R^{3a} each independently represent H or an alkyl group,
R^{4a} represents H, a halogen, or an organic group,
X^{1a} represents a halogen other than F, and
X² represents a halogen,
the method comprising step 1a of allowing a compound represented by formula (1):

   R¹O- Y⁺ (1)
wherein
   Y⁺ represents a cation and R¹ is as defined above,
   to react, in the presence of a halogenating agent, with a compound represented by formula (2a): 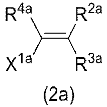
   wherein symbols in the formula are as defined above.

### [Embodiment 2]

The production method according to Embodiment 1, wherein the halogenating agent is a compound represented by formula (3a):

X²-Z (3a)

wherein
X² represents a halogen,
Z represents a halogen or NZ¹Z²,
Z¹ and Z² each independently represent an organic group, or Z¹ and
Z² are bound together to form a ring, or
a compound represented by formula (3a'): 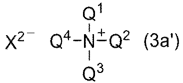 wherein
   X² represents a halogen,
   Q¹ to Q⁴ each independently represent H or an organic group, and
   any two of Q¹ to Q⁴ are optionally bound together to form a ring.

### [Embodiment 3]

The production method according to Embodiment 1 or 2, wherein Y⁺ represents a metal ion or a quaternary ammonium ion.

### [Embodiment 4]

The production method according to any one of Embodiments 1 to 3, wherein R¹ represents a fluoroalkyl group optionally having one or more substituents, and the fluoroalkyl group optionally contains a heteroatom between carbon atoms.

### [Embodiment 5]

The production method according to any one of Embodiments 1 to 4, wherein R¹ represents a C₁₋C₁₀ fluoroalkyl group optionally having one or more substituents, and the C₁₋C₁₀ fluoroalkyl group optionally contains a heteroatom between carbon atoms.

### [Embodiment 6]

The production method according to any one of Embodiments 1 to 5, wherein R^{4a} is F or a fluoroalkyl group.

### [Embodiment 7]

The production method according to any one of Embodiments 1 to 6, wherein R^{2a} and R^{3a} each independently represent H or a C₁₋C₃ alkyl group.

### [Embodiment 8]

The production method according to any one of Embodiments 1 to 7, wherein step 1a is performed in the absence of a catalyst.

### [Embodiment 9]

A method for producing a compound represented by formula (4b): 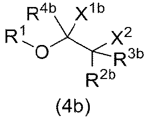 wherein
R¹ represents an organic group,
R^{2b} and R^{3b} each independently represent H, a halogen, or an alkyl group optionally having one or more substituents,
R^{4b} represents H, a halogen, or an organic group, and
X^{1b} and X² each independently represent a halogen,
the method comprising step 1b of allowing a compound represented by formula (1):

   R¹O⁻ Y⁺ (1)
wherein
   Y⁺ represents a cation and R¹ is as defined above,
   to react, in the presence of a halogenating agent and a catalyst,
   with a compound represented by formula (2b): 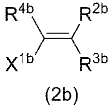
   wherein symbols in the formula are as defined above.

### [Embodiment 10]

The production method according to Embodiment 9, wherein when R^{4b} represents F or a fluoroalkyl group, X^{1b} represents a halogen other than F.

### [Embodiment 11]

The production method according to Embodiment 9 or 10, wherein the halogenating agent is a compound represented by formula (3a):

X²-Z (3a)

wherein
X² represents a halogen,
Z represents a halogen or NZ¹Z²,
Z¹ and Z² each independently represent an organic group, or Z¹ and
Z² are bound together to form a ring, or
a compound represented by formula (3a'): 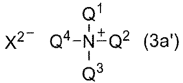 wherein
   X² represents a halogen,
   Q¹ to Q⁴ each independently represent H or an organic group, and any two of Q¹ to Q⁴ are optionally bound together to form a ring.

### [Embodiment 12]

The production method according to any one of Embodiments 9 to 11, wherein the catalyst is a Lewis base.

### [Embodiment 13]

The production method according to any one of Embodiments 9 to 12, wherein the catalyst is a Lewis base represented by formula (3b):

L¹- (Z³) ₙ (3b)

wherein
L¹ represents O, N, (S)ₘ, P, or (Se)ₖ,
m and k are 1 or 2,
n represents a number corresponding to the valence of L¹,
Z³s each independently represent an organic group, and when n represents 2 or more, any two Z³s are optionally bound together to form a ring,
or a Lewis base represented by formula (3c):

   L²=Z⁴ (3c)

   wherein
   L² represents O, S, or Se,
   Z⁴ represents P(Z⁵)₃ or C(Z⁶)₂, Z⁵s each independently represent an organic group, and any two Z⁵s are optionally bound together to form a ring, and Z⁶s each independently represent an organic group, and any two Z⁶s are optionally bound together to form a ring.

### [Embodiment 14]

The production method according to any one of Embodiments 9 to 13, wherein Y⁺ represents a metal ion or a quaternary ammonium ion.

### [Embodiment 15]

The production method according to any one of Embodiments 9 to 14, wherein R¹ represents a fluoroalkyl group optionally having one or more substituents, and the fluoroalkyl group optionally contains a heteroatom between carbon atoms.

### [Embodiment 16]

The production method according to any one of Embodiments 9 to 15, wherein R^{2b} and R^{3b} each independently represent H, a halogen, an alkyl group, or a fluoroalkyl group.

### [Embodiment 17]

The production method according to any one of Embodiments 9 to 16, wherein R^{4b} represents a halogen or a fluoroalkyl group.

### [Embodiment 18]

A compound represented by formula (4a): 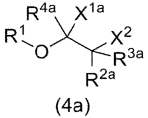 wherein
R¹ represents an organic group,
R^{2a} and R^{3a} each independently represent H or an alkyl group,
R^{4a} represents H, a halogen, or an organic group,
X^{1a} represents a halogen other than F, and
X² represents a halogen.

### [Embodiment 19]

A composition comprising a compound represented by formula (4b): 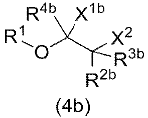 wherein
R¹ represents an organic group,
R^{2b} and R^{3b} each independently represent H, a halogen, or an alkyl group optionally having one or more substituents,
R^{4b} represents H, a halogen, or an organic group,
X^{1b} and X² each independently represent a halogen; and
a halide ion.

### [Embodiment 20]

A composition comprising a compound represented by formula (4b): 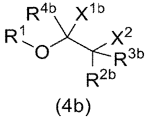 wherein
R¹ represents an organic group,
R^{2b} and R^{3b} each independently represent H, a halogen, or an alkyl group optionally having one or more substituents,
R^{4b} represents H, a halogen, or an organic group, and
X^{1b} and X² each independently represent a halogen; and water.

### [Embodiment 21]

The composition according to Embodiment 19 or 20, wherein when R^{4b} represents F or a fluoroalkyl group, X^{1b} represents a halogen other than F.

### [Embodiment 22]

A method for producing a compound represented by formula (5a): 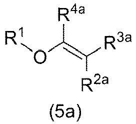 wherein
R¹ represents an organic group,
R^{2a} and R^{3a} each independently represent H or an alkyl group, R^{4a} represents H, a halogen, or an organic group,
the method comprising step 2a of allowing a reducing agent to react with a compound represented by formula (4a): 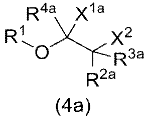 wherein
   R¹, R^{2a}, R^{3a}, and R^{4a} are as defined above,
   X^{1a} represents a halogen other than F, and
   X² represents a halogen.

### [Embodiment 23]

The production method according to Embodiment 22, wherein R¹ represents a fluoroalkyl group optionally having one or more substituents, and the fluoroalkyl group optionally contains a heteroatom between carbon atoms.

### [Embodiment 24]

The production method according to Embodiment 22 or 23, wherein R¹ represents a C₁₋C₁₀ fluoroalkyl group optionally having one or more substituents, and the C₁₋C₁₀ fluoroalkyl group optionally contains a heteroatom between carbon atoms.

### [Embodiment 25]

The production method according to any one of Embodiments 22 to 24, wherein R^{4a} represents F or a fluoroalkyl group.

### [Embodiment 26]

The production method according to any one of Embodiments 22 to 25, wherein the reducing agent is an organometallic reagent.

### [Embodiment 27]

The production method according to any one of Embodiments 22 to 25,
wherein the reducing agent is at least one member selected from the group consisting of
(i) transition metals, alkali metals, and alkaline earth metals;
(ii) metal pairs of transition metals; and
(iii) mixtures of transition metals with acids or metal salts.

### [Embodiment 28]

The production method according to any one of Embodiments 22 to 25, wherein the reducing agent is a phosphorus-containing compound.

### [Embodiment 29]

A method for producing a compound represented by formula (5a): 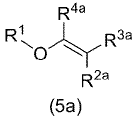 wherein
R¹ represents an organic group,
R^{2a} and R^{3a} each independently represent H or an alkyl group, and
R^{4a} represents H, a halogen, or an organic group,
the method comprising a step of producing a compound represented by formula (4a): 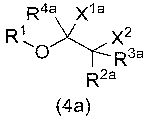 wherein
   R¹, R^{2a}, R^{3a}, R^{4a}, and x^{1a} are as defined above, and
   X² represents a halogen, comprising
   step 1a of allowing a compound represented by formula (1):

      R¹O⁻ Y⁺ (1)

      wherein
      Y⁺ represents a cation and R¹ is as defined above, to react, in the presence of a halogenating agent, with a compound represented by formula (2a): 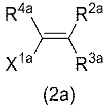 wherein
      R^{2a}, R^{3a}, and R^{4a} are as defined above,
      X^{1a} represents a halogen other than F, and step 2a of allowing a reducing agent to react with the compound represented by formula (4a).

### [Embodiment 30]

A method for producing a compound represented by formula (5b): 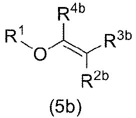 wherein
R¹ represents an organic group,
R^{2b} and R^{3b} each independently represent H, a halogen, or an alkyl group optionally having one or more substituents, and
R^{4b} represents H, a halogen, or an organic group,
the method comprising a step of producing a compound represented by formula (4b): 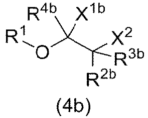 wherein
   R¹, R^{2b}, R^{3b}, R^{4b}, and X^{1b} are as defined above, and
   X² represents a halogen, comprising
   step (1b) of allowing a compound represented by formula (1): R

      ¹O⁻ Y⁺ (1)

      wherein
      Y⁺ represents a cation and R¹ is as defined above,
      to react, in the presence of a halogenating agent and a catalyst, with a compound represented by formula (2b): 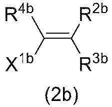 wherein
         R^{2b}, R^{3b}, and R^{4b} are as defined above, and
         X^{1b} represents a halogen, and
         step 2b of allowing a reducing agent to react with the compound represented by formula (4b).

### [Embodiment 31]

The production method according to Embodiment 30, wherein when R^{4b} represents F or a fluoroalkyl group, X^{1b} represents a halogen other than F.

### [Embodiment 32]

A compound represented by formula (5c): 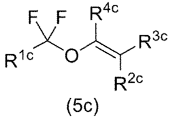 wherein
R^{1c} represents a fluoroalkyl group having two or more carbon atoms and optionally having one or more substituents, or a fluoroalkoxy group having one or more carbon atoms and optionally having one or more substituents, wherein the fluoroalkyl group or the fluoroalkoxy group optionally contains a heteroatom between carbon atoms,
R^{2c} and R^{3c} each independently represent H or an alkyl group, and R^{4c} represents F.

### [Embodiment 33]

A composition comprising a compound represented by formula (5b): 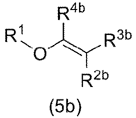 wherein
R¹ represents an organic group,
R^{2b} and R^{3b} each independently represent H, a halogen, or an alkyl group optionally having one or more substituents,
R^{4b} represents H, a halogen, or an organic group; and
a halide ion.

### [Embodiment 34]

A composition comprising a compound represented by formula (5b): 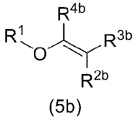 wherein
R¹ represents an organic group,
R^{2b} and R^{3b} each independently represent H, a halogen, or an alkyl group optionally having one or more substituents,
R^{4b} represents H, a halogen, or an organic group; and water.

## Claims

1. A compound of formula (5c): 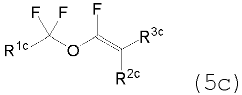 wherein
R^{1c} is C_{≥2}-fluoroalkyl or C_{≥1}-fluoroalkoxy, each optionally containing a heteroatom selected from oxygen atom, sulfur atom, and nitrogen atom between carbon atoms,
(I) R^{2c} and R^{3c} are both H or (II) R^{2c} is H and R^{3c} is C₁₋₃-alkyl.

2. A composition comprising
(i) a compound of formula (5b): 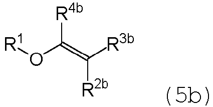 wherein
R¹ is fluoroalkyl which (i) optionally has one or more substituents and (ii) optionally contains a heteroatom selected from oxygen atom, sulfur atom, and nitrogen atom between carbon atoms,
R^{2b}, R^{3b} each independently are H, halogen, or alkyl,
R^{4b} is H, halogen, or fluoroalkyl; and
(ii) per 100 mass% of the composition, ≤ 10 mass% of a halide ion, determined by ion chromatography.
